(19) Europäisches Patentamt
European Patent Office
Office européen des brevets

(11) **EP 2 205 277 B1**

# (12) EUROPEAN PATENT SPECIFICATION

(45) Date of publication and mention
of the grant of the patent:
**26.07.2017 Bulletin 2017/30**

(51) Int Cl.:
**A61K 39/395** (2006.01)    **C07K 16/28** (2006.01)

(21) Application number: **08841962.7**

(22) Date of filing: **22.10.2008**

(86) International application number:
**PCT/DK2008/050256**

(87) International publication number:
**WO 2009/052830 (30.04.2009 Gazette 2009/18)**

(54) **NOVEL ANTIBODY THERAPIES**

NEUARTIGE ANTIKÖRPERTHERAPIEN

NOUVELLES THÉRAPIES D'ANTICORPS

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **22.10.2007 DK 200701519**

(43) Date of publication of application:
**14.07.2010 Bulletin 2010/28**

(73) Proprietor: **Genmab A/S**
**1260 Copenhagen K (DK)**

(72) Inventors:
- **GOLAB, Jakub**
**02-122 Warsaw (PL)**
- **WINIARSKA, Magdalena**
**01-651 Warsaw (PL)**
- **PARREN, Paul**
**3984 PR Odijk (NL)**
- **MACKUS, Wendy**
**3522 SB Utrecht (NL)**
- **ENGELBERTS, Patrick**
**3813 MR Amersfoort (NL)**

(74) Representative: **Genmab A/S**
**Kalvebod Brygge 43**
**1560 Copenhagen V (DK)**

(56) References cited:
- **CRAGG M S ET AL: "The biology of CD20 and its potential as a target for mAb therapy" CURRENT DIRECTIONS IN AUTOIMMUNITY, KARGER, BASEL, CH, 1 January 2005 (2005-01-01), pages 140-174, XP008087727 ISSN: 1422-2132**
- **UNRUH TAMMY L ET AL: "Cholesterol depletion inhibits src family kinase-dependent calcium mobilization and apoptosis induced by rituximab crosslinking" IMMUNOLOGY, vol. 116, no. 2, October 2005 (2005-10), pages 223-232, XP002517983 ISSN: 0019-2805**
- **POLYAK M J ET AL: "A cholesterol-dependent CD20 epitope detected by the FMC7 antibody." LEUKEMIA (BASINGSTOKE), vol. 17, no. 7, July 2003 (2003-07), pages 1384-1389, XP002517984 ISSN: 0887-6924**
- **TEELING JESSICA L ET AL: "The biological activity of human CD20 monoclonal antibodies is linked to unique epitopes on CD20" JOURNAL OF IMMUNOLOGY, AMERICAN ASSOCIATION OF IMMUNOLOGISTS, US, vol. 177, no. 1, 1 July 2006 (2006-07-01), pages 362-371, XP002453762 ISSN: 0022-1767**
- **WINIARSKA MAGDALENA ET AL: "Statins impair antitumor effects of rituximab by inducing conformational changes of CD20." PLOS MEDICINE 25 MAR 2008, vol. 5, no. 3, 25 March 2008 (2008-03-25), page e64, XP009113180 ISSN: 1549-1676**

EP 2 205 277 B1

SEQUENCE LISTING

<110> Genmab A/S

EP 2 205 277 B1

Medical University of Warsaw

<120> Novel Antibody Therapies

<130> P/45.WO

<160> 15

<170> PatentIn version 3.3

<210> 1
<211> 122
<212> PRT
<213> homo sapiens

<400> 1

```
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
        50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser
            115                 120
```

<210> 2
<211> 107
<212> PRT
<213> homo sapiens

<400> 2

```
Glu Ile Val Leu Thr Gln Ser Pro Ala Thr Leu Ser Leu Ser Pro Gly
1               5                   10                  15
```

**24**

```
        Glu Arg Ala Thr Leu Ser Cys Arg Ala Ser Gln Ser Val Ser Ser Tyr
                    20                  25                  30

        Leu Ala Trp Tyr Gln Gln Lys Pro Gly Gln Ala Pro Arg Leu Leu Ile
                    35                  40                  45

        Tyr Asp Ala Ser Asn Arg Ala Thr Gly Ile Pro Ala Arg Phe Ser Gly
                50                  55                  60

        Ser Gly Ser Gly Thr Asp Phe Thr Leu Thr Ile Ser Ser Leu Glu Pro
        65                  70                  75                  80

        Glu Asp Phe Ala Val Tyr Tyr Cys Gln Gln Arg Ser Asn Trp Pro Ile
                        85                  90                  95

        Thr Phe Gly Gln Gly Thr Arg Leu Glu Ile Lys
                    100                 105
```

<210> 3
<211> 5
<212> PRT
<213> homo sapiens

<400> 3
Asp Tyr Ala Met His 1 5

<210> 4
<211> 17
<212> PRT
<213> homo sapiens

<400> 4

```
        Thr Ile Ser Trp Asn Ser Gly Ser Ile Gly Tyr Ala Asp Ser Val Lys
        1               5                   10                  15

        Gly
```

<210> 5
<211> 13
<212> PRT
<213> homo sapiens

<400> 5

```
            Asp Ile Gln Tyr Gly Asn Tyr Tyr Tyr Gly Met Asp Val
            1               5                   10
```

<210> 6
<211> 11
<212> PRT
<213> homo sapiens

<400> 6

```
                    Arg Ala Ser Gln Ser Val Ser Ser Tyr Leu Ala
                    1               5                   10
```

<210> 7
<211> 7
<212> PRT
<213> homo sapiens

<400> 7

```
                         Asp Ala Ser Asn Arg Ala Thr
                         1               5
```

<210> 8
<211> 9
<212> PRT
<213> homo sapiens

<400> 8

```
                    Gln Gln Arg Ser Asn Trp Pro Ile Thr
                    1               5
```

<210> 9
<211> 17
<212> PRT
<213> homo sapiens

<400> 9

```
          Asp Tyr Tyr Gly Ala Gly Ser Phe Tyr Asp Gly Leu Tyr Gly Met Asp
          1               5                   10                  15

          Val
```

<210> 10
<211> 81
<212> PRT
<213> homo sapiens

<400> 10

```
          Asp Tyr Ala Met His Trp Val Arg Gln Ala Pro Gly Lys Gly Leu Glu
          1               5                   10                  15

          Trp Val Ser Thr Ile Ser Trp Asn Ser Gly Ser Ile Gly Tyr Ala Asp
                      20                  25                  30

          Ser Val Lys Gly Arg Phe Thr Ile Ser Arg Asp Asn Ala Lys Lys Ser
                      35                  40                  45
```

```
Leu Tyr Leu Gln Met Asn Ser Leu Arg Ala Glu Asp Thr Ala Leu Tyr
    50                  55                  60
```

```
Tyr Cys Ala Lys Asp Ile Gln Tyr Gly Asn Tyr Tyr Tyr Gly Met Asp
    65              70                  75                  80
```

```
Val
```

<210> 11
<211> 14
<212> PRT
<213> homo sapiens

<400> 11

```
Asp Asn Gln Tyr Gly Ser Gly Ser Thr Tyr Gly Leu Gly Val
1               5                   10
```

<210> 12
<211> 125
<212> PRT
<213> homo sapiens

<400> 12

```
Glu Val Gln Leu Val Gln Ser Gly Gly Gly Leu Val His Pro Gly Gly
1               5                   10                  15
```

```
Ser Leu Arg Leu Ser Cys Ala Gly Ser Gly Phe Thr Phe Ser Ser Tyr
            20                  25                  30
```

```
Ala Met His Trp Val Arg Gln Ala Pro Gly Lys Gly Leu Glu Trp Val
        35                  40                  45
```

```
Ser Ala Ile Gly Thr Gly Gly Gly Thr Tyr Tyr Ala Asp Ser Val Lys
    50                  55                  60
```

```
Gly Arg Phe Thr Ile Ser Arg Asp Asn Ala Lys Asn Ser Leu Tyr Leu
    65                  70                  75                  80
```

```
Gln Met Asn Ser Leu Arg Ala Glu Asp Met Ala Val Tyr Tyr Cys Ala
                85                  90                  95
```

```
Arg Asp Tyr Tyr Gly Ser Gly Ser Tyr Tyr Tyr Tyr Tyr Tyr Gly Met
            100                 105                 110
```

```
Asp Val Trp Gly Gln Gly Thr Thr Val Thr Val Ser Ser
            115                 120                 125
```

<210> 13

<211> 107
<212> PRT
<213> homo sapiens

<400> 13

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
            35                  40                  45

Tyr Asp Ala Ser Asn Arg Ala Thr Gly Ile Pro Ala Arg Phe Ser Gly
            50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Leu
                85                  90                  95

Thr Phe Gly Gly Gly Thr Lys Val Glu Ile Lys
            100                 105
```

<210> 14
<211> 122
<212> PRT
<213> homo sapiens

<400> 14

```
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
            35                  40                  45

Ser Gly Ile Ser Trp Asn Ser Gly Ser Ile Gly Tyr Ala Asp Ser Val
            50                  55                  60

Lys Gly Arg Phe Thr Ile Ser Arg Asp Asn Ala Lys Asn Ser Leu Tyr
65                  70                  75                  80
```

```
        Leu Gln Met Asn Ser Leu Arg Ala Glu Asp Thr Ala Leu Tyr Tyr Cys
                        85              90                      95

        Ala Lys Asp Ile Asp Tyr Tyr Tyr Tyr Tyr Gly Met Asp Val Trp
                        100             105             110

        Gly Gln Gly Thr Thr Val Thr Val Ser Ser
                        115             120
```

<210> 15
<211> 107
<212> PRT
<213> homo sapiens

<400> 15

```
        Glu Ile Val Leu Thr Gln Ser Pro Ala Thr Leu Ser Leu Ser Pro Gly
        1               5               10              15

        Glu Arg Ala Thr Leu Ser Cys Arg Ala Ser Gln Ser Val Ser Ser Tyr
                        20              25              30

        Leu Ala Trp Tyr Gln Gln Lys Pro Gly Gln Ala Pro Arg Leu Leu Ile
                        35              40              45

        Tyr Asp Ala Ser Asn Arg Ala Thr Gly Ile Pro Ala Arg Phe Ser Gly
                50              55              60

        Ser Gly Ser Gly Thr Asp Phe Thr Leu Thr Ile Ser Ser Leu Glu Pro
        65              70              75              80

        Glu Asp Phe Ala Val Tyr Tyr Cys Gln Gln Arg Ser Asn Trp Pro Ile
                        85              90              95

        Thr Phe Gly Gln Gly Thr Arg Leu Glu Ile Lys
                        100             105
```

**Description**

**FIELD OF THE INVENTION**

The present invention relates to the use of an antibody capable of mediating CDC, which specifically binds to CD20, and which comprises specific CDR sequences for use in the treatment of a disease or disorder associated with said antigen, wherein the disease or disorder is selected from B cell lymphoma, B cell leukemia or an autoimmune disease, and wherein antibody-induced CDC has a beneficial effect on said disease or disorder, wherein the antibody is to be administered to a subject undergoing therapy with a cholesterol-lowering agent, such as a statin, and wherein the subject is withdrawn from treatment with the cholesterol-lowering agent prior to the administration of the antibody.

**BACKGROUND OF THE I NVENTI ON**

Antibodies are being used as therapeutic agents for a number of diseases and disorders, including cancer and autoimmune diseases. Antibodies are immunoglobulins that recognize specific antigens and mediate their effects via several mechanisms, including inhibition of ligand-receptor interactions, inhibition of receptor activation, mediation of receptor internalization and activation of effector functions, such as complement dependent cytotoxicity (CDC) and antibody-dependent cellular cytotoxicity (ADCC). There are five classes of immunoglobulins: IgG, IgA, IgM, IgD and IgE. The IgG class is further divided into subclasses IgG1, IgG2, IgG3 and IgG4.

Polyak MJ et al. (2003) Leukemia, 17:1384-1389 disclose that cholesterol depletion reduced expression of the epitope of CD20 to which the anti-CD20 antibody FCM7 binds, whereas cholesterol enrichment enhanced its expression. It should be noted that the FMC7 antigen is still poorly characterized even though binding of anti-FMC7 monoclonal antibody is critically dependent on the presence of CD20 in the plasma membrane. Therefore, any conformational changes in CD20 might result in impaired binding to FMC7 antigen.

Janas E et al. (2005) Clinical and Experimental Immunology, 139:439-446 show that the integrity of lipid rafts seem to play a crucial role for CD20-induced calcium influx and induction of apoptosis, and that depletin of cholesterol with M$\beta$CD profoundly reduces apoptosis induced by Rituxan®-mediated cross-linking of CD20.

Cragg MS et al. (2005) Curr Dir Autoimmun, 8:140-174 disclose the results of testing the effect of cholesterol depletion (with MCD) and cholesterol enrichment, respectively, on the binding affinity of various anti-CD20 antibodies, including the FMC7 antibody. The authors conclude that the effect of cholesterol depletion is dependent on the particular antibody and cell line used, some of the antibodies being relatively unaffected. FMC7 is the most sensitive. In the experiment loading the cells with cholesterol prior to antibody binding only FMC7 shows a positive effect.

The present invention provides new regimens for an anti-CD20 antibody defined by specific CDR sequences based on the finding that depletion of cholesterol decreases effector function induced by the anti-CD20 antibody, whereas enrichment with cholesterol increases effector function. By increasing the serum cholesterol the effector function induced by the antibody will increase thereby resulting in a higher efficacy of the antibody. The invention will therefore be useful for the present therapeutic anti-CD20 antibody capable of mediating effector function, wherein effector function is significantly or mainly contributing to the therapeutic effect.

Other features and advantages of the instant invention will be apparent from the following detailed description and examples which should not be construed as limiting.

**DEFINITIONS**

The terms "CD20" and "CD20 antigen" are used interchangeably herein, and include any variants, isoforms and species homologs of human CD20, which are naturally expressed by cells or are expressed on cells transfected with the CD20 gene. Synonyms of CD20, as recognized in the art, include B-lymphocyte surface antigen B1, Leu-16 and Bp35. Human CD20 has UniProtKB/Swiss-Prot entry P11836.

The term "immunoglobulin" as used herein refers to a class of structurally related glycoproteins consisting of two pairs of polypeptide chains, one pair of light (L) low molecular weight chains and one pair of heavy (H) chains, all four inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as $V_H$) and a heavy chain constant region. The heavy chain constant region, $C_H$, typically is comprised of three domains, $C_H1$, $C_H2$, and $C_H3$. Each light chain typically is comprised of a light chain variable region (abbreviated herein as $V_L$) and a light chain constant region. The light chain constant region typically is comprised of one domain, $C_L$. The $V_H$ and $V_L$ regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs).

Each $V_H$ and $V_L$ is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901-917 (1987)). Typically, the numbering of amino acid residues in this region is performed by the method described in Kabat et al., Sequences of Proteins of Immunological Interest, 5th Ed. Public Health Service, National Institutes of Health, Bethesda, MD. (1991) (phrases, such as variable domain residue numbering as in Kabat or according to Kabat herein refer to this numbering system for heavy chain variable domains or light chain variable domains). Using this numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (for instance residue 52a according to Kabat) after residue 52 of $V_H$ CDR2 and inserted residues (for instance residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The term "antibody" as used herein refers to an immunoglobulin molecule, a fragment of an immunoglobulin molecule, or a derivative of either thereof, which has the ability to specifically bind to an antigen under typical physiological conditions for a significant period of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or a time sufficient for the antibody to recruit an Fc-mediated effector activity).

The term "anti-CD20 antibody" as used herein refer to any molecule that specifically binds to a portion of CD20 under cellular and/or physiological conditions for an amount of time sufficient to inhibit the activity of CD20 expressing cells and/or otherwise modulate a physiological effect associated with CD20; to allow detection by ELISA, western blot, or other similarly suitable binding technique described herein and/or known in the art and/or to otherwise be detectably bound thereto after a relevant period of time (for instance at least about 15 minutes, such as at least about 30 minutes, at least about 45 minutes, at least about 1 hour, at least about 2 hours, at least about 4 hours, at least about 6 hours, at least about 12 hours, such as about 1-24 hours, about 1-36 hours, about 1-48 hours, about 1-72 hours, about one week, or longer).

The variable regions of the heavy and light chains of the immunoglobulin molecule contain a binding domain that interacts with an antigen. The constant regions of the antibody may mediate the binding of the immunoglobulin to host tissues or factors, including various cells of the immune system (such as effector cells) and components of the complement system such as C1q, the first component in the classical pathway of complement activation.

The antibody may be mono-, bi- or multispecific.

As indicated above, the term "antibody" as used herein, unless otherwise stated or clearly contradicted by the context, includes fragments of an antibody provided by any known technique, such as enzymatic cleavage, peptide synthesis and recombinant techniques that retain the ability to specifically bind to an antigen. It has been shown that the antigen-binding function of an antibody may be performed by fragments of a full-length (intact) antibody. Examples of antigen-binding fragments encompassed within the term "antibody" include, but are not limited to (i) a Fab fragment, a monovalent fragment consisting of the $V_L$, $V_H$, $C_L$ and $C_H1$ domains; (ii) F(ab)₂ and F(ab')₂ fragments, bivalent fragments comprising two Fab fragments linked by a disulfide bridge at the hinge region; (iii) a Fd fragment consisting essentially of the $V_H$ and $C_H1$ domains; (iv) a Fv fragment consisting essentially of the $V_L$ and $V_H$ domains of a single arm of an antibody, (v) a dAb fragment (Ward et al., Nature 341, 544-546 (1989)), which consists essentially of a $V_H$ domain and also called domain antibodies (Holt et al. (November 2003) Trends Biotechnol. 21 (11):484-90) ; (vi) a camelid antibody or nanobody (Revets et al. (January 2005) Expert Opin Biol Ther. 5(1):111-24), (vii) an isolated complementarity determining region (CDR), such as a $V_H$ CDR3, (viii) a UniBody® molecule, a monovalent antibody as disclosed in WO 2007/059782, (ix) a single chain antibody or single chain Fv (scFv), see for instance Bird et al., Science 242, 423-426 (1988) and Huston et al., PNAS USA 85, 5879-5883 (1988)), (x) a diabody (a scFv dimer), which can be monospecific or bispecific (see for instance PNAS USA 90(14), 6444-6448 (1993), EP 404097 or WO 93/11161 for a description of diabodies), a triabody or a tetrabody.

Although such fragments are generally included within the definition of an antibody, they collectively and each independently are unique features of the present invention, exhibiting different biological properties and utility. These and other useful antibody fragments in the context of the present invention are discussed further herein.

As used herein, "specific binding" refers to the binding of a binding molecule, such as a full-length antibody or an antigen-binding fragment thereof, to a predetermined antigen. Typically, the antibody binds with an affinity corresponding to a $K_D$ of about $10^{-7}$ M or less, such as about $10^{-8}$ M or less, such as about $10^{-9}$ M or less, about $10^{-10}$ M or less, or about $10^{-11}$ M or even less, when measured for instance using sulfon plasmon resonance on BIAcore or as apparent affinities based on $IC_{50}$ values in FACS or ELISA, and binds to the predetermined antigen with an affinity corresponding to a $K_D$ that is at least ten-fold lower, such as at least 100 fold lower, for instance at least 1000 fold lower, such as at least 10,000 fold lower, for instance at least 100,000 fold lower than its affinity for binding to a non-specific antigen (e.g., BSA, casein) other than the predetermined antigen or a closely-related antigen. When the $K_D$ of the antigen binding peptide is very low (that is, the antigen binding peptide is highly specific), then the affinity for the antigen may be at least 10,000 or 100,000 fold lower than the affinity for a non-specific antigen.

It should be understood that the term antibody generally includes monoclonal antibodies as well as polyclonal antibodies. The antibodies can be human, humanized, chimeric, murine, etc. An antibody as generated can possess any isotype.

The term "human antibody", as used herein, is intended to include antibodies having variable and constant regions derived from human germline immunoglobulin sequences. The human antibodies of the present invention may include amino acid residues not encoded by human germline immunoglobulin sequences (for instance mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted into human framework sequences.

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, a human antibody derived from a particular human germline sequence will display no more than 10 amino acid differences, such as no more than 5, for instance no more than 4, 3, 2, or 1 amino acid difference from the amino acid sequence encoded by the germline immunoglobulin gene. For $V_H$ antibody sequences the $V_H$ CDR3 domain is not included in such comparison.

The term "chimeric antibody" refers to an antibody that contains one or more regions from one antibody and one or more regions from one or more other antibodies. The term "chimeric antibody" includes monovalent, divalent, or polyvalent antibodies. A monovalent chimeric antibody is a dimer (HL) formed by a chimeric H chain associated through disulfide bridges with a chimeric L chain. A divalent chimeric antibody is a tetramer ($H_2L_2$) formed by two HL dimers associated through at least one disulfide bridge. A polyvalent chimeric antibody may also be produced, for example, by employing a CH region that assembles into a molecule with 2+ binding sites (for instance from an IgM H chain, or $\mu$ chain). Typically, a chimeric antibody refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see for instance US 4,816,567 and Morrison et al., PNAS USA 81, 6851-6855 (1984)). Chimeric antibodies are produced by recombinant processes well known in the art (see for instance Cabilly et al., PNAS USA 81, 3273-3277 (1984), Morrison et al., PNAS USA 81, 6851-6855 (1984), Boulianne et al., Nature 312, 643-646 (1984), EP125023, Neuberger et al., Nature 314, 268-270 (1985), EP171496, EP173494, WO 86/01533, EP184187, Sahagan et al., J. Immunol. 137, 1066-1074 (1986), WO 87/02671, Liu et al., PNAS USA 84, 3439-3443 (1987), Sun et al., PNAS USA 84, 214-218 (1987), Better et al., Science 240, 1041-1043 (1988) and Harlow et al., Antibodies: A Laboratory Manual, Cold Spring Harbor Laboratory Press, Cold Spring Harbor, N.Y., (1988)).

The term "humanized antibody" refers to a human antibody which contains minimal sequences derived from a non-human antibody. Typically, humanized antibodies are human immunoglobulins (recipient antibody) in which residues from a hypervariable region of the recipient are replaced by residues from a hypervariable region of a non-human species (donor antibody), such as mouse, rat, rabbit or non-human primate having the desired specificity, affinity, and capacity.

Furthermore, humanized antibodies may comprise residues which are not found in the recipient antibody or in the donor antibody. These modifications are made to further refine antibody performance. In general, a humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. A humanized antibody optionally also will comprise at least a portion of a human immunoglobulin constant region. For further details, see Jones et al., Nature 321, 522-525 (1986), Riechmann et al., Nature 332, 323-329 (1988) and Presta, Curr. Op. Struct. Biol. 2, 593-596 (1992).

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be generated by a hybridoma which includes a B cell obtained from a transgenic or transchromosomal nonhuman animal, such as a transgenic mouse, having a genome comprising a human heavy chain transgene and a light chain transgene, fused to an immortalized cell.

The term "recombinant human antibody", as used herein, includes all human antibodies that are prepared, expressed, created or isolated by recombinant means, such as (a) antibodies isolated from an animal (such as a mouse) that is transgenic or transchromosomal for human immunoglobulin genes or a hybridoma prepared therefrom (described further elsewhere herein), (b) antibodies isolated from a host cell transformed to express the antibody, such as from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies may be subjected to *in vitro* mutagenesis (or, when an animal transgenic for human Ig sequences is used, *in vivo* somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire *in vivo*.

The terms "transgenic, non-human animal" refers to a non-human animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain transgene or human heavy chain transchromosome, such that the mouse produces human anti-CD20 antibodies when immunized with CD20 antigen and/or cells expressing CD20. The human heavy chain transgene may be integrated into the chromosomal DNA of the mouse, as is the case for transgenic mice, for instance the HuMAb-Mouse®, such as HCo7 or HCo12 mice as described in in detail in Taylor L et al. (1992) Nucleic Acids Research 20:6287-6295; Chen J et al. (1993) International Immunology 5:647-656; Tuaillon et al. (1994) J. Immunol. 152:2912-2920; Lonberg N et al. (1994) Nature 368(6474):856-859; Lonberg N (1994) Handbook of Experimental Pharmacology 113:49-101; Taylor L et al. (1994) International Immunology 6: 579-591; Lonberg N and Huszar D (1995) Intern. Rev. Immunol. Vol. 13:65-93; Harding F and Lonberg N (1995) Ann. N.Y. Acad. Sci 764:536-546; Fishwild D et al. (1996) Nature Biotechnology 14:845-851. See further, US Nos. 5,545,806; 5,569,825; 5,625,126; 5,633,425; 5,789,650; 5,877,397; 5,661,016; 5,814,318; 5,874,299; and 5,770,429; all to Lonberg and Kay, as well as US 5,545,807 to Surani et al.; WO 98/24884, WO 94/25585, WO 93/1227, WO 92/22645, WO 92/03918 and WO 01/09187, or the human heavy chain transgene may be maintained extrachromosomally, as is the case for the transchromosomal KM-Mouse® as described in WO 02/43478. Such transgenic and transchromosomal mice (collectively referred to herein as "transgenic mice") are capable of producing multiple isotypes of human monoclonal antibodies to a given antigen (such as IgG, IgA, IgM, IgD and/or IgE) by undergoing V-D-J recombination and isotype switching. Transgenic, nonhuman animals can also be used for production of antibodies against a specific antigen by introducing genes encoding such specific antibody, for example by operatively linking the genes to a gene which is expressed in the milk of the animal.

The term "treatment" as used herein means the administration of an effective amount of a therapeutically active compound of the present invention with the purpose of easing, ameliorating, arresting, or eradicating (curing) symptoms or disease states.

## SEQUENCE LISTING

| SEQ ID NO:1 | 2F2 $V_H$ | EVQLVESGGGLVQPGRSLRLSCAASGFTFNDYAMHWVRQAPGKGLEWVSTISWNSGSIGYADSVKGRFTISRDNAKKSLYLQMNSLRAEDTALYYCAKDIQYGNYYYGMDVWGQGTTVTVSS |
|---|---|---|
| SEQ ID NO:2 | 2F2 $V_L$ | EIVLTQSPATLSLSPGERATLSCRASQSVSSYLAWYQQKPGQAPRLLIYDASNRATGIPARFSGSGSGTDFTLTISSLEPEDFAVYYCQQRSNWPITFGQGTRLEIK |
| SEQ ID NO:3 | 2F2 $V_H$ CDR1 | DYAMH |
| SEQ ID NO:4 | 2F2 $V_H$ CDR2 | TISWNSGSIGYADSVKG |

(continued)

| SEQ ID NO:5 | 2F2 V$_H$ CDR3 | DIQYGNYYYGMDV |
|---|---|---|
| SEQ ID NO:6 | 2F2VL CDR1 | RASQSVSSYLA |
| SEQ ID NO:7 | 2F2 V$_L$ CDR2 | DASNRAT |
| SEQ ID NO:8 | 2F2 V$_L$ CDR3 | QQRSNWPIT |
| SEQ ID NO:9 | 11B8 V$_H$ CDR3 | DYYGAGSFYDGLYGMDV |
| SEQ ID NO:10 | 2F2 V$_H$ CDR1-CDR3 | DYAMHWVRQAPGKGLEWVSTISWNSGSIGYADSVKGRFTISRDNAKKSLYLQMNSLRAEDTALYYCAKDIQYGNYYYGMDV |
| SEQ ID NO:11 | 2C6 V$_H$ CDR3 | DNQYGSGSTYGLGV |
| SEQ ID NO:12 | Human V$_H$ DP-44/D3-10/JH6b germline sequence | EVQLVQSGGGLVHPGGSLRLSCAGSGFTFSSYAMHWVRQAPGKGLEWVSAIGTGGGTYYADSVKGRFTISRDNAKNSLYLQMNSLRAEDMAVYYCARDYYGSGSYYYYYYGMDVWGQGTTVTVSS |
| SEQ ID NO:13 | Human V$_L$ L6/JK4 germline sequence | EIVLTQSPATLSLSPGERATLSCRASQSVSSYLAWYQQKPGQAPRLLIYDASNRATGIPARFSGSGSGTDFTLTISSLEPEDFAVYYCQQRSNWPLTFGGGTKVEIK |
| SEQ ID NO:14 | Human V$_H$ 3-09/D4-11/JH6b germline sequence | EVQLVESGGGLVQPGRSLRLSCAASGFTFDDYAMHWVRQAPGKGLEWVSGISWNSGSIGYADSVKGRFTISRDNAKNSLYLQMNSLRAEDTALYYCAKDIDYYYYYYGMDVWGQGTTVTVSS |
| SEQ ID NO:15 | Human V$_L$ L6/JK5 germline sequence | EIVLTQSPATLSLSPGERATLSCRASQSVSSYLAWYQQKPGQAPRLLIYDASNRATGIPARFSGSGSGTDFTLTISSLEPEDFAVYYCQQRSNWPITFGQGTRLEIK |

**BRIEF DESCRIPTION OF THE DRAWINGS**

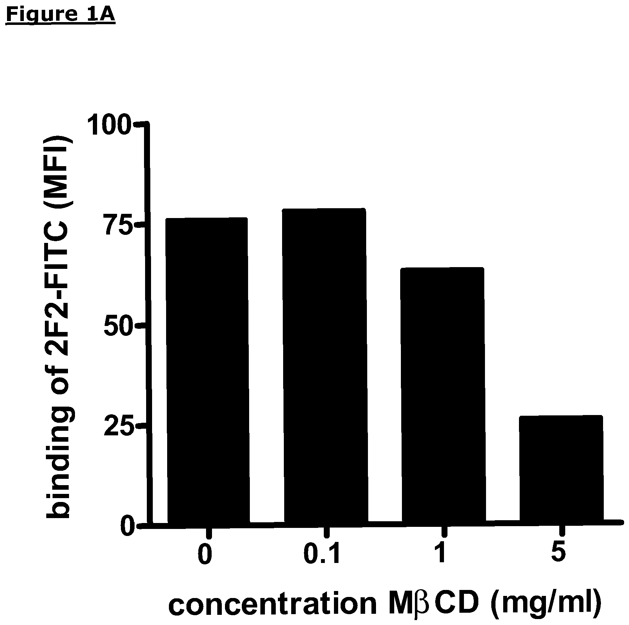

*Figure 1A* shows a dose-dependent decrease in binding of FITC-conjugated anti-CD20 mAb 2F2 to Daudi cells treated with varying concentrations of MβCD as measured by mean fluorescence intensity (MFI).

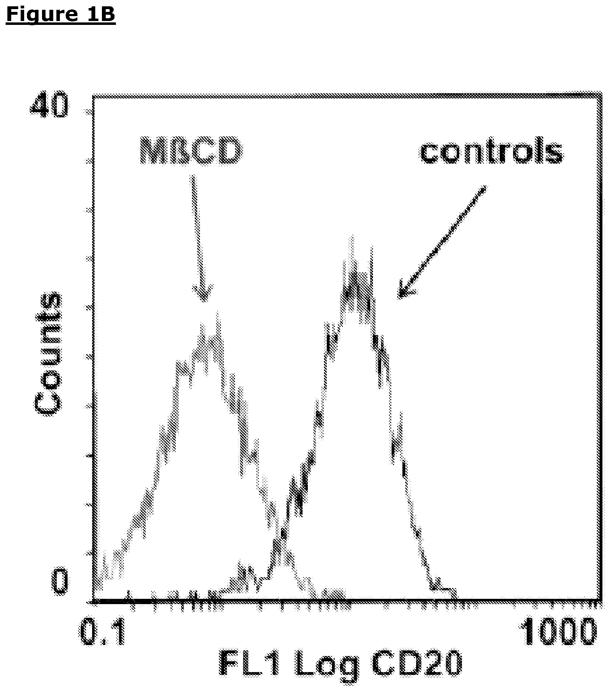

*Figure 1B* shows a decreased binding of FITC-conjugated anti-CD20 mAb B9E9 in RAJI-CD20high cells incubated with 5 mg/ml MβCD in comparison with non-treated cells as measured by flow cytometry.

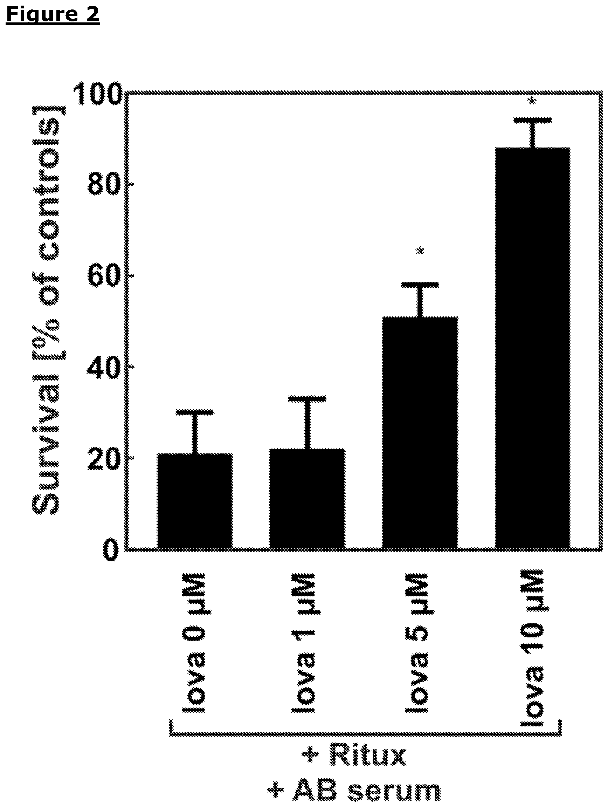

*Figure 2* shows a dose-dependent protection of RAJI-CD20high cells from rituximab-mediated CDC at varying concentrations of the statin, lovastatin as determined in a MTT assay.

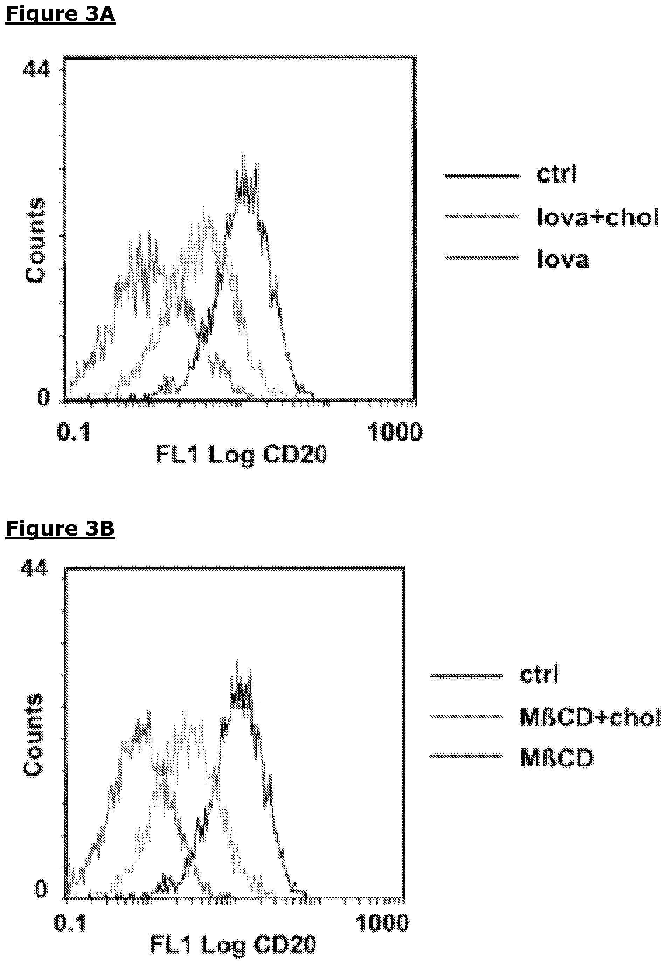

*Figure 3A* shows that incubation of RAJI-CD20high cells with cholesterol upon pre-treatment with lovastatin reinstates binding of B9E9.

*Figure 3B* shows that that incubation of RAJI-CD20high cells with cholesterol upon pre-treatment with MβCD reinstates binding of B9E9.

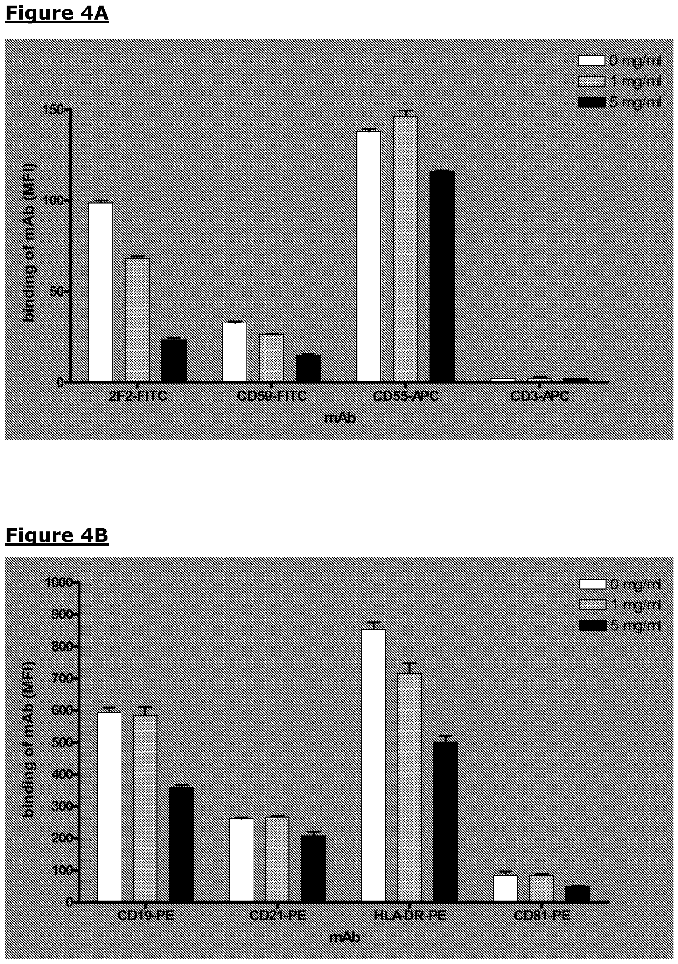

*Figure 4A* and *Figure 4B* show FACS analysis of binding of mAb to various cell surface proteins on RAJI cells incubated with increasing concentrations of MβCD. Data are presented as mean $\pm$ SEM of two independent observations. Binding is expressed as mean fluorescence intensity (MFI).

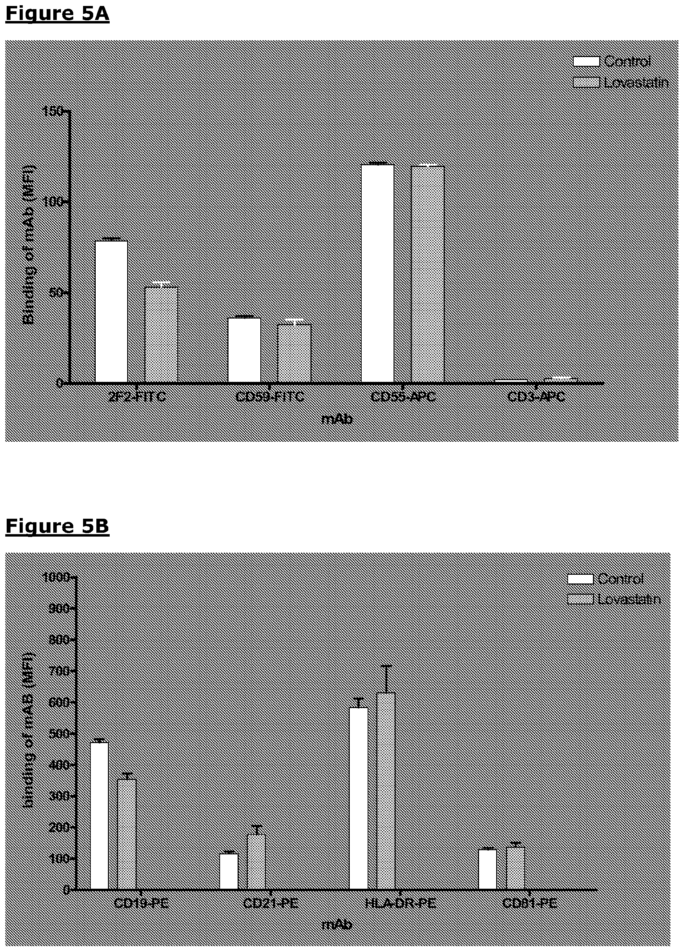

*Figure 5A* and *Figure 5B* show FACS analysis of binding of various mAb to various cell surface proteins on RAJI cells incubated with increasing concentrations of lovastatin. Data are presented as mean $\pm$ SEM of two independent observations. Binding is expressed as mean fluorescence intensity (MFI).

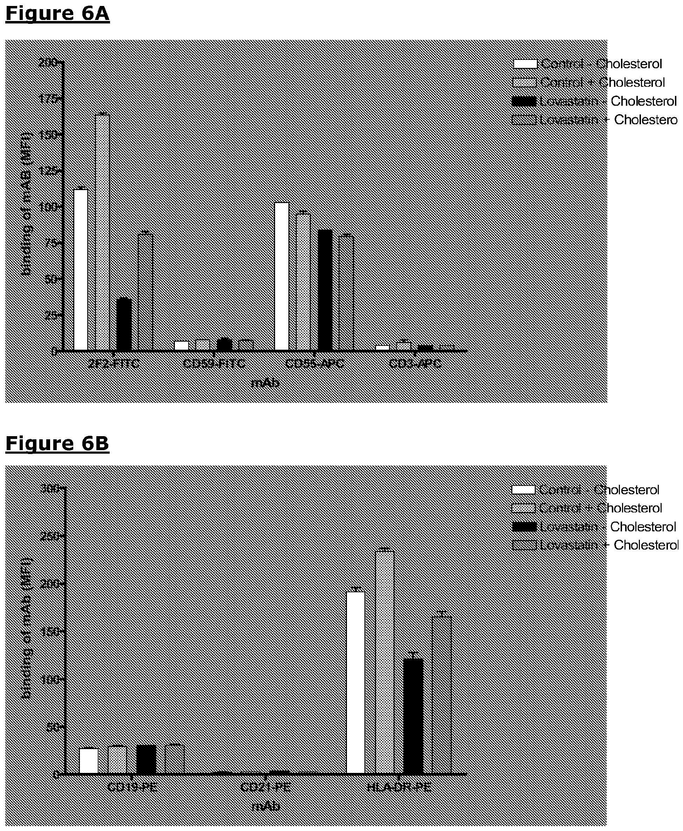

*Figure 6A* and *Figure 6B* show FACS analysis of binding of various mAb to various cell surface proteins on WIL2-S cells incubated with increasing concentrations of lovastatin. Data are presented as mean $\pm$ SEM of two independent observations. Binding is expressed as mean fluorescence intensity (MFI).

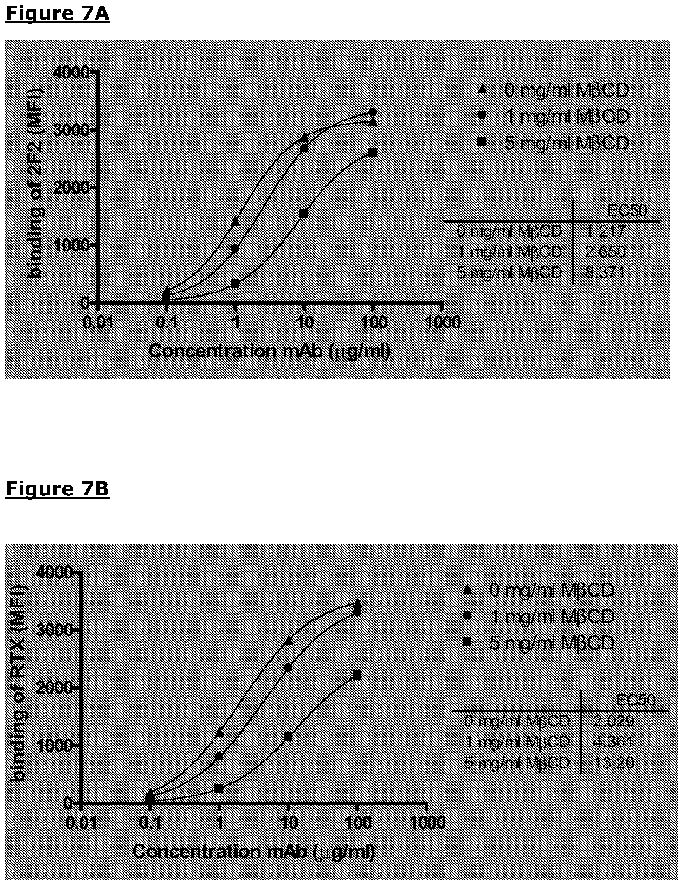

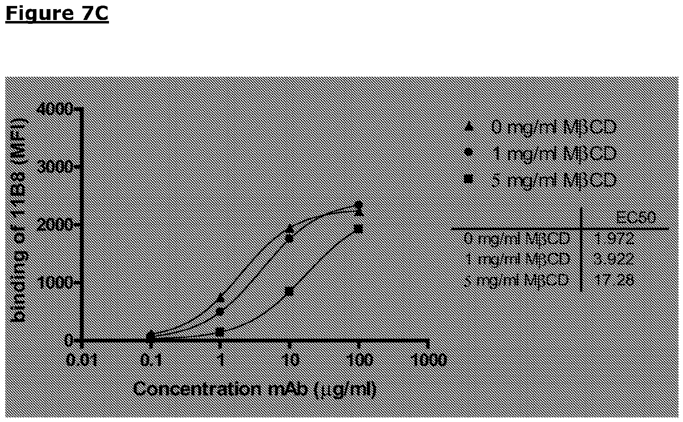

*Figure 7* shows the binding of 2F2 (A), rituximab (B) and 11 B8 (C) to Daudi cells incubated with various concentrations of MβCD. Mean fluorescence intensity (MFI) is shown as measure for binding intensity of 2F2 to cells treated with varying concentrations of MβCD. Data are presented as mean $\pm$ SEM of two independent observations. The concentrations of $EC_{50}$ are determined from a four-parameter logistic curve fit and expressed in μg/ml.

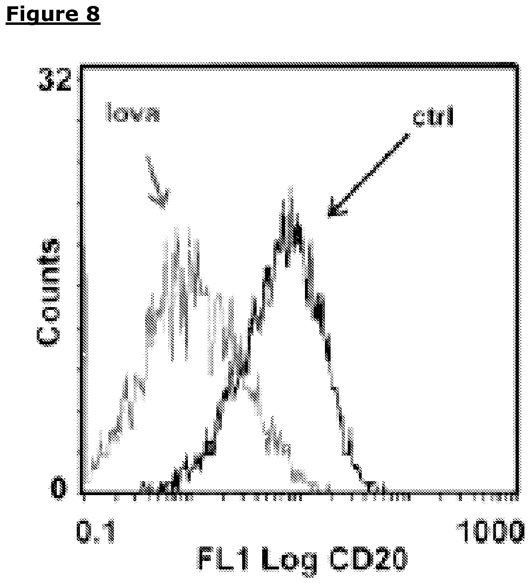

*Figure 8* shows binding of B9E9 on RAJI-CD20high cells incubated with 10 μM lovastatin in comparison with non-treated cells as measured by flow cytometry.

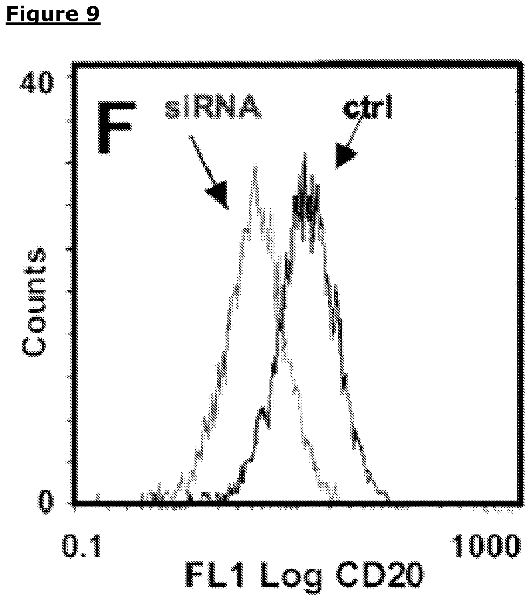

*Figure 9* shows the influence of siRNA targeting HMG-CoAR on mAb-mediated CD20 detection. RAJI cells were transfected with siRNA targeting HMG-CoAR or irrelevant siRNA using oligofectAMINE™. After 48 hours, $1 \times 10^6$/ml of cells were incubated with saturating amounts of FITC-conjugated B9E9 or IgG1 (isotypic control) for 30 minutes at room temperature in the dark. On the Y-axis the number of positive stained cells indicated by counts is shown, and on the X-axis the staining intensity is shown.

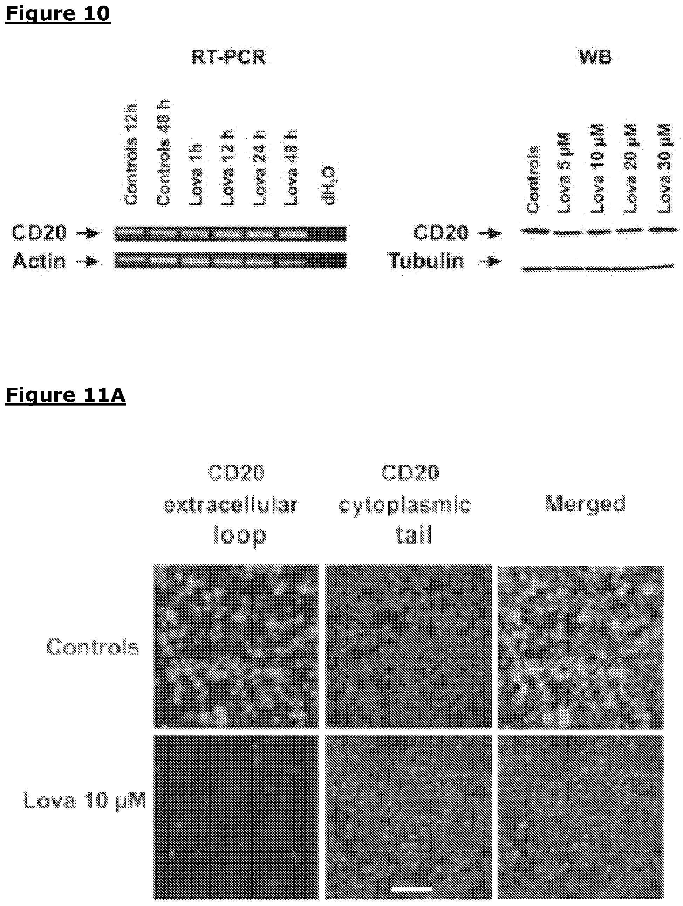

*Figure 10, left panel shows* the CD20 mRNA levels in RAJI-CD20high cells incubated with either diluent (control) or 10 μM lovastatin for 1 to 48 hours assayed by RT-PCR. Actin mRNA levels were determined as reference for loading controls. *Right panel* shows the CD20 protein levels in RAJI-CD20high cells incubated with either diluent or 5, 10, 20, and 30 μM lovastatin for 48 hours assayed by Western blotting.

*Figure 11A* shows the result of immunofluorescence studies of RAJI-CD20high cells incubated for 48 hours with either diluent or 10 μM lovastatin. RAJI-CD20high cells were incubated with FITC-conjugated (light grey) B9E9 to detect the extracellular (conformational) epitope located in the larger CD20 loop. Upon washing with PBS, the cells were permeablized with acetone and incubated with IgG1 antibody (NCL-CD20-L26) directed against a cytoplasmic (linear) epitope within the CD20 molecule. After washing, a secondary Alexa555-labeled (dark grey) anti-IgG1 (anti-NCL-CD20-L26) antibody was used. Bar = 50 μm.

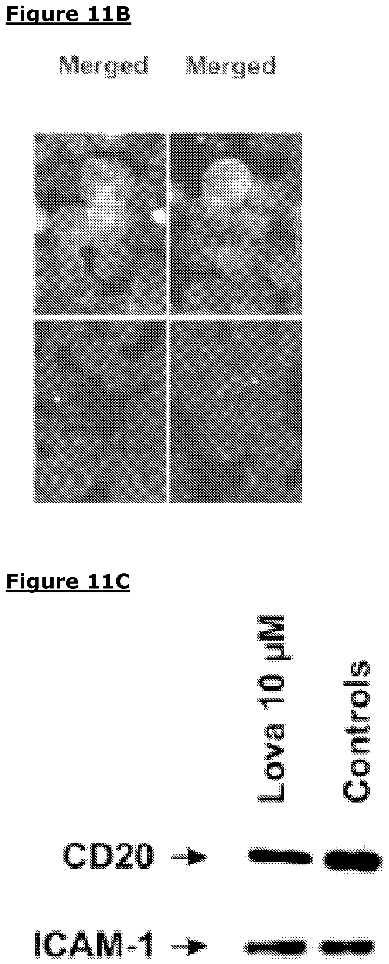

*Figure 11B* shows a higher magnification of RAJI-CD20 high cells stained with both extracellular and cytoplasmic epitopes (two different areas are shown for each group, stars indicate a membrane localization).

*Figure 11C* shows control cells or lovastatin-treated RAJI cells (10 μM for 48 hours) ($1 \times 10^6$) incubated with EZ-link® Sulfo-NHS-biotin. Total cellular lysates were precipitated with immobilized NeutrAvidin protein followed by electrophoresis and blotting with anti-CD20 and anti-ICAM-1 mAbs.

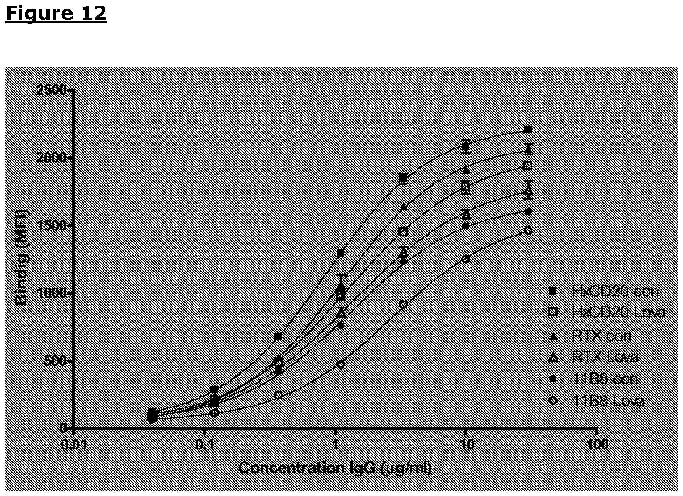

*Figure 12* shows mean fluorescence intensity (MFI) as a measure for binding intensity of anti-CD20 mAbs 2F2, rituximab and 11 B8 to cells treated with 10 μM lovastatin (Lova, open symbols) compared to non-treated cells (con; closed symbols). Data are presented as mean $\pm$ SEM of two independent observations.

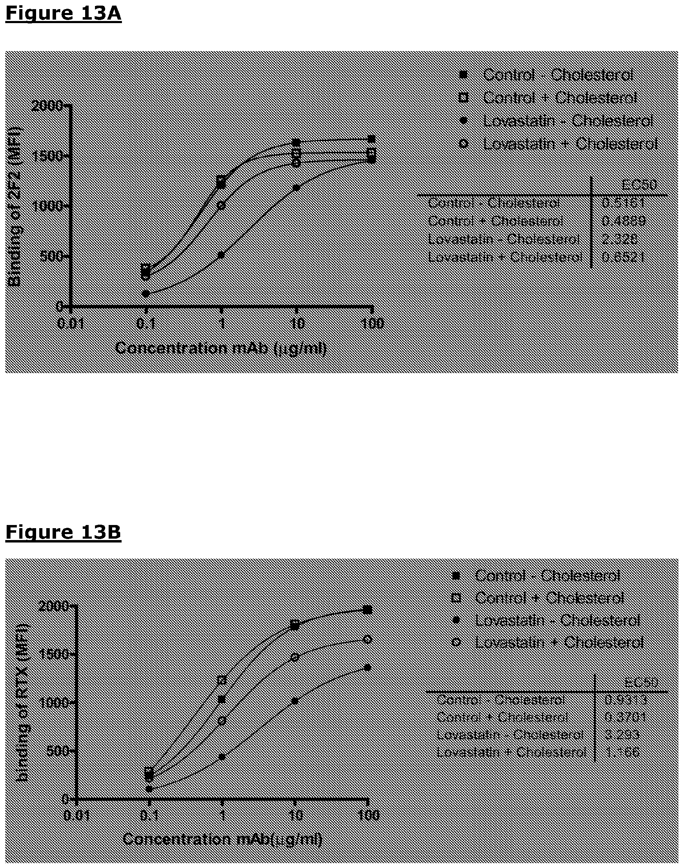

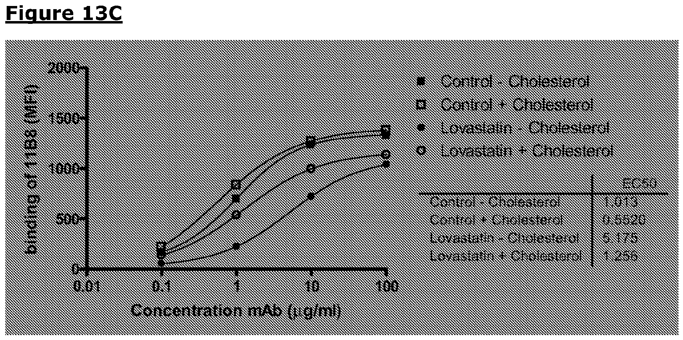

*Figure 13* shows binding of 2F2 (A), rituximab (B) or 11 B8 (C) to WIL2-S cells cultured in the presence of lovastatin as cholesterol depleting agent, and replenished with cholesterol. Data are presented as mean $\pm$ SEM of two independent observations. The $EC_{50}$ values are determined from a four-parameter logistic curve fit and expressed in μg/ml.

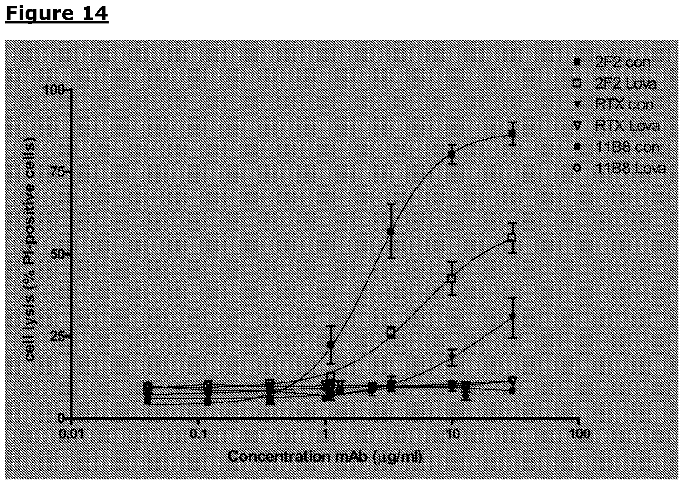

*Figure 14* shows lysis of lovastatin-treated Daudi cells via CDC induced by 2F2, rituximab and 11 B8. Data are presented as mean $\pm$ SEM of two independent observations. Analysis was performed by staining of lysed cells using PI and flow cytometry.

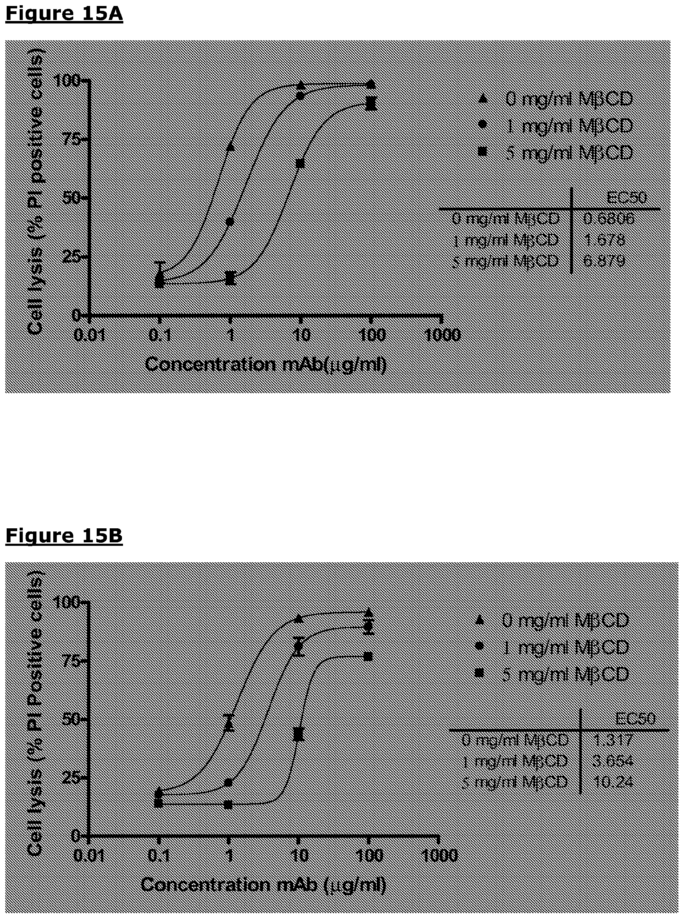

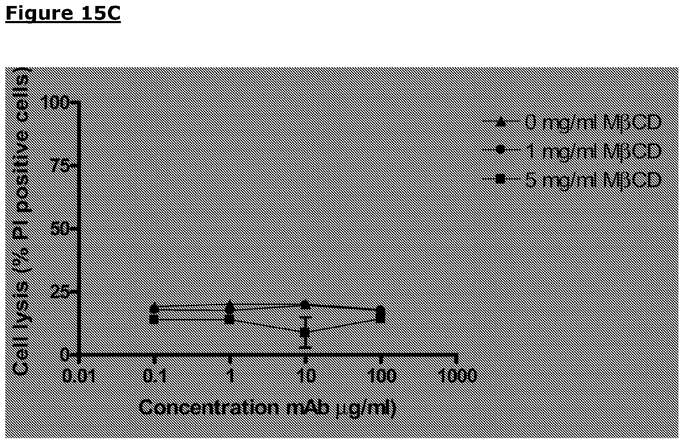

*Figure 15* shows lysis of MβCD-treated Daudi cells via CDC induced by 2F2 (A), rituximab (B) and 11 B8 (C). Data are presented as mean $\pm$ SEM of two independent observations. Analysis was performed by staining of lysed cells using PI and flow cytometry. The $EC_{50}$ values are determined from a four-parameter logistic curve fit and expressed in μg/ml.

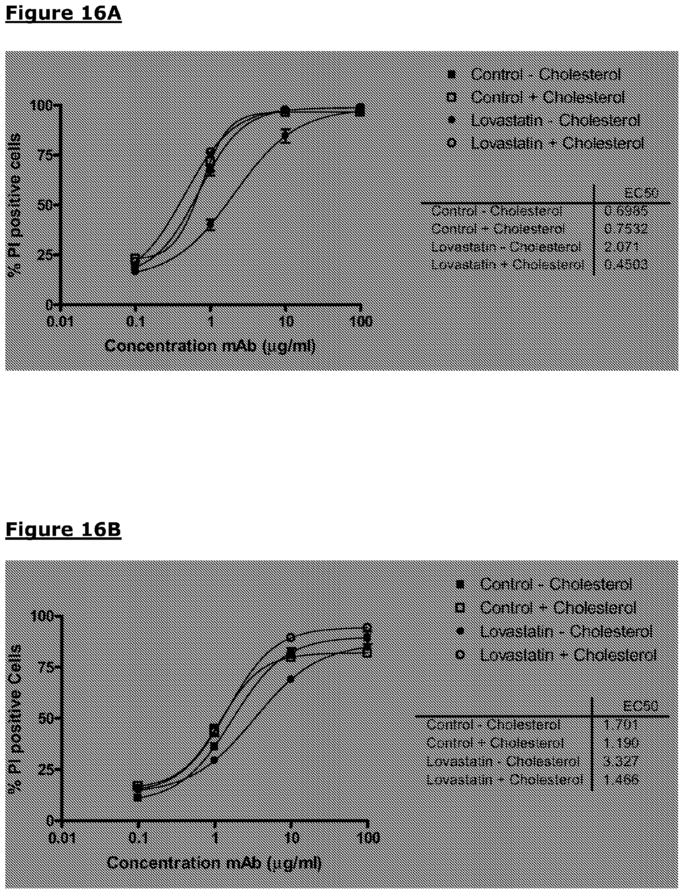

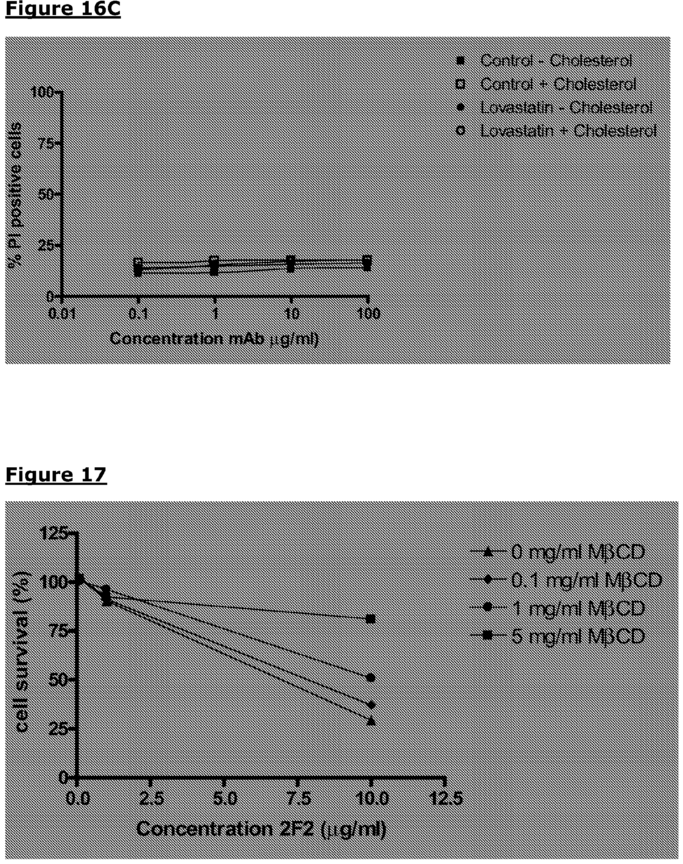

*Figure 16* shows anti-CD20 mAb-induced CDC-mediated lysis of WIL2-S cells with varing cholesterol cell membrane contents. Data show induction of CDC by 2F2 (A), rituximab (B) and 11 B8 (C). The $EC_{50}$ values are determined from a four-parameter logistic curve fit and expressed in μg/ml.

*Figure 17* shows 2F2-induced CDC of Daudi cells incubated with various concentrations of MβCD. Cell survival is detected using the MTT assay and expressed as percentage of survival compared to control.

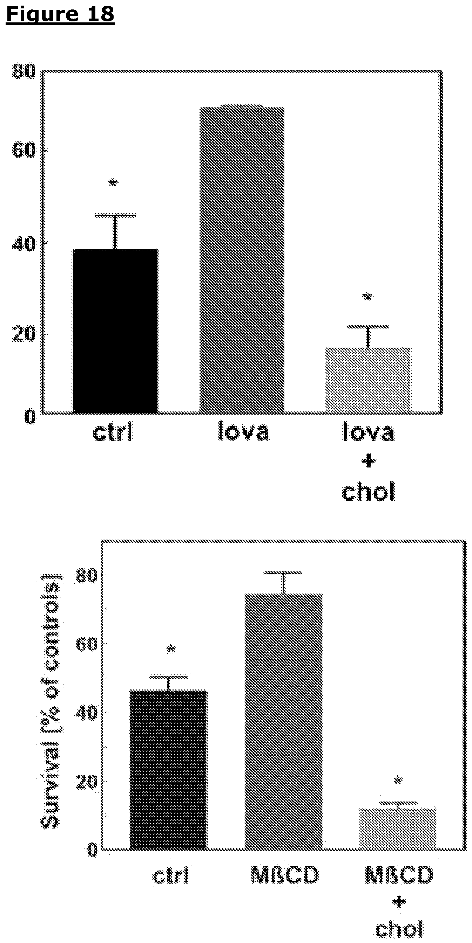

*Figure 18* shows MTT analysis of rituximab-induced CDC of RAJI cells depleted for cholesterol via lovastatin (left figure, drak grey bar) or MβCD (right figure, dark grey bar) and reconstituted with cholesterol (light grey bars) in comparison with non-treated cell (control, black bars). Data are presented as mean ± SEM of at least n=3 observations.

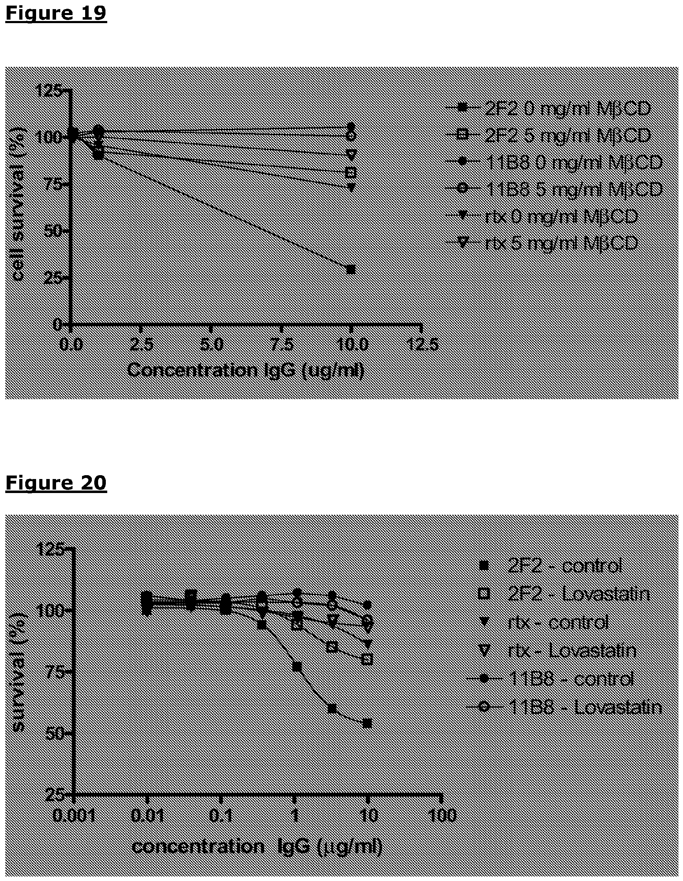

*Figure 19* shows MTT analysis of anti-CD20 mAb-induced CDC of RAJI cells incubated with various concentrations of MβCD as cholesterol depleting agent. Data show ability of 2F2 (square) and rituximab (rtx triangle) to decrease survival of RAJI cells upon incubation with MβCD (open symbols) or without (closed symbols) upon depletion of cholesterol. Upon incubation with 11B8 no CDC (circle) is induced (negative control).

*Figure 20* shows anti-CD20 mAb-induced CDC of Daudi cells depleted for cholesterol using lovastatin as detected using the Alamar Blue assay. Data show ability of 2F2 (square) and rituximab (rtx, triangle) to decrease survival of Daudi cells upon incubation with lovastatin (open symbols) or without lovastatin (closed symbols). Upon incubation with 11 B8 no CDC (circle) is induced (negative control).

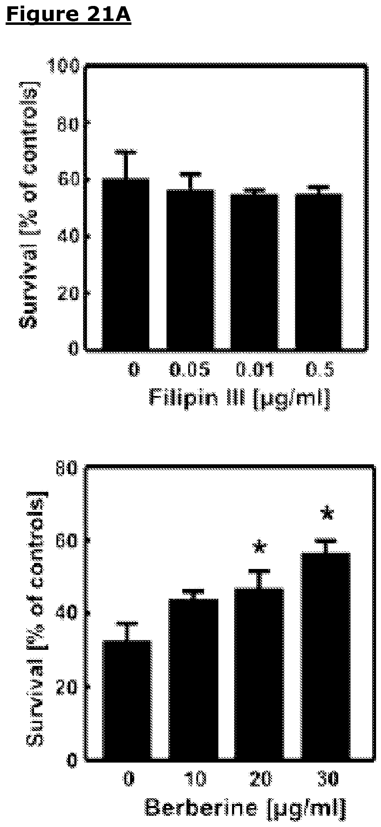

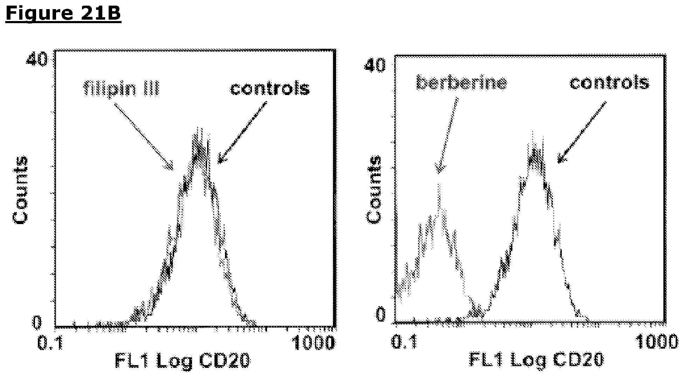

*Figure 21* shows the influence of cholesterol-modulating drugs on the expression of CD20 in RAJI-CD20high cells. (A) RAJI-CD20high cells were pre-incubated with either diluents or a concentration range of Fillipin III for 30 minutes (left figure) or a concentration range of Berberine chloride (right figure) for 24 hours. Pre-incubated cells were subsequently incubated with 10 μg/ml rituximab and 10% complement-active serum for 1 hour upon which the percentage of viable cells was detected using a MTT assay. Data show mean and SEM of three independent experiments. * P< 0.05 is considered statistically significant (2-way Student's t-test) as compared to controls. (B) Diluent-treated cells (control; dark) or Fillipin III-treated cells (left figure) or Berberine chloride-treated cells (right figure) were incubated with saturating amounts of FITC-conjugated B9E9 and mAb binding was detected using flow cytometry. The binding intensity was expressed as mean fluorescence intensity (MFI).

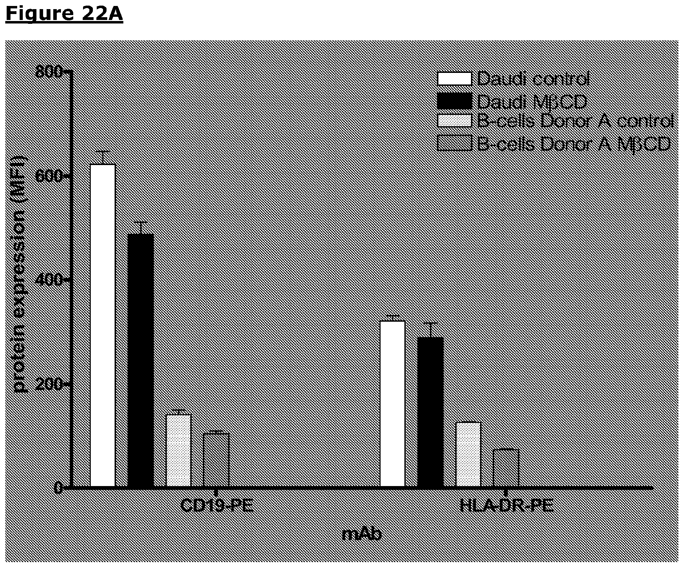

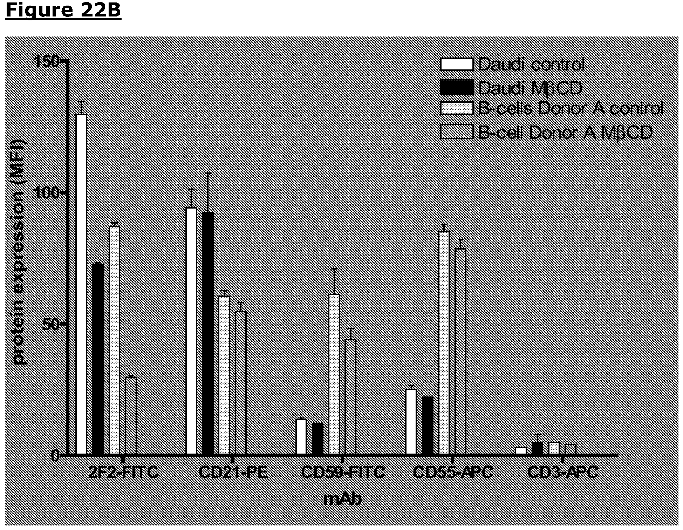

*Figure 22* shows detection of cell surface proteins on cholesterol-depleted primary B cells. The B cell enriched cell population of a healthy volunteer (donor A) was incubated with MβCD to deplete cholesterol. Daudi cells was included as a control. As expression of B cell markers such as CD20, CD19 and CD21 was determined as well as of complement regulatory proteins CD55 and CD59 by staining with mouse monoclonal mAb (A & B). CD20 was detected using FITC-labeled 2F2. Data represent protein expression as expressed by mean fluorescence intensity (MFI). Data show mean and SD of a duplicate measurement (n= 1).

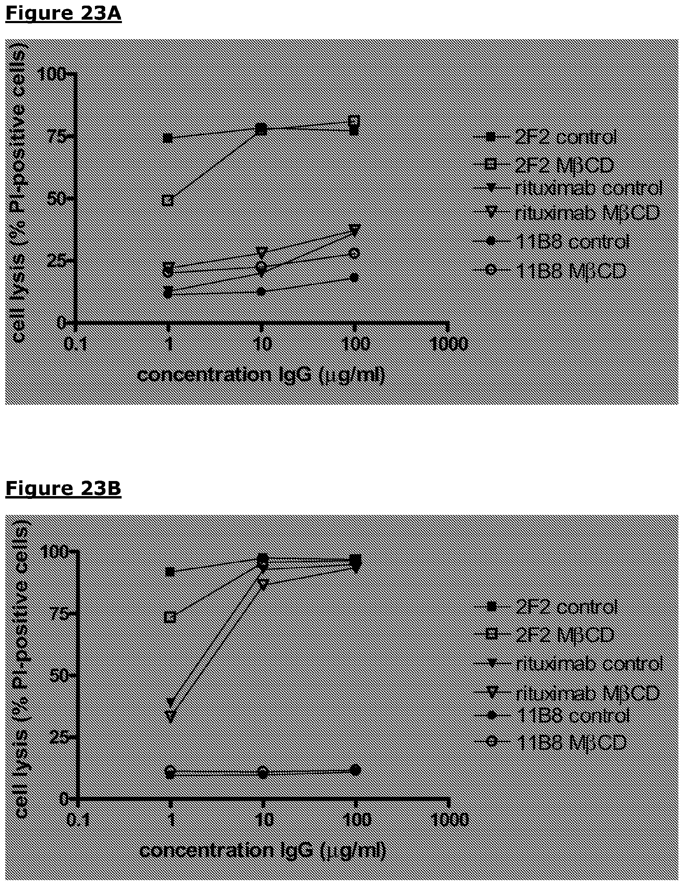

*Figure 23* shows anti-CD20-induced CDC of cholesterol-depleted primary B cells (A) and Daudi cells (B). CDC was induced by incubating MβCD- or non-treated B cell enriched cell population of a healthy volunteer (Donor A) (A) or Daudi cells (B) with either 2F2 (square), rituximab (triangle) or 11 B8 (circle) and normal human serum as complement source (15% final concentration). Data represent duplicate staining of a single experiment. Cell lysis was expressed as percentage of PI-positive cells as determined using flow cytometry.

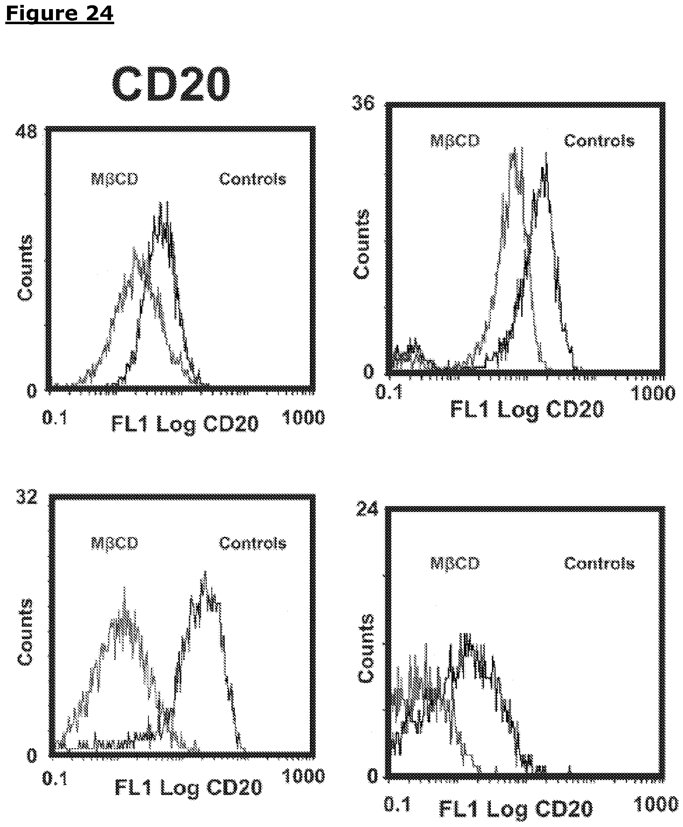

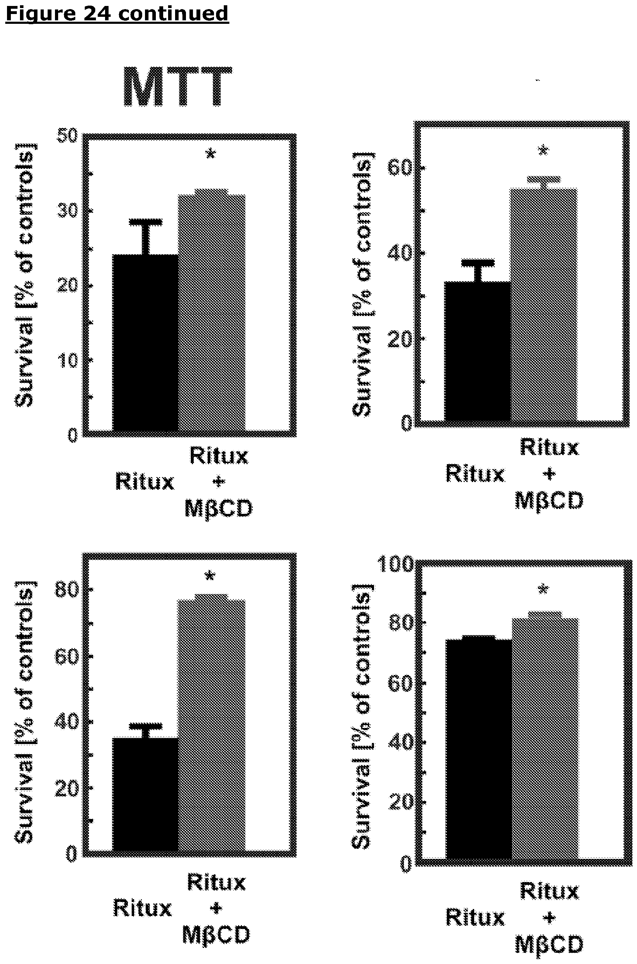

*Figure 24* shows the influence of cholesterol depletion by MβCD on the expression of CD20 as well as on rituximab-mediated CDC against freshly isolated human lymphoma cells. Primary human CD20+ B cell lymphoma cells (mantle cell lymphomas in P1, P2, P3, and small B cell lymphoma in P4) were incubated with either diluent (black) or MβCD (10 mg/ml, grey) for 30 minutes. Then, $1 \times 10^6$ cells/ml were incubated with saturating amounts of FITC-conjugated B9E9 for 30 minutes at room temperature in the dark (left panel). CD20 mAb binding was measured using a flow cytometer. For the rituximab-mediated CDC human (right panel) CD20+ B cell lymphoma cells were incubated with either diluent or 10 mg/ml MβCD for 30 minutes. Then, equal numbers of cells ($1 \times 10^5$/well) were incubated for 60 minutes with 400 μg/ml rituximab in the presence of 10% normal human serum as a source of complement. The cytotoxic effects were measured in a MTT assay. The survival of cells is presented as % of corresponding diluent- or MβCD-pretreated cells without rituximab. * *P* < 0.05 (two-way Student's *t*-test) as compared to controls.

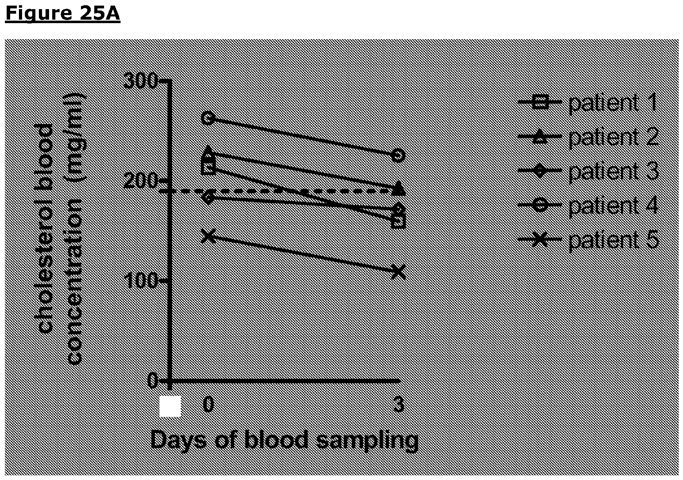

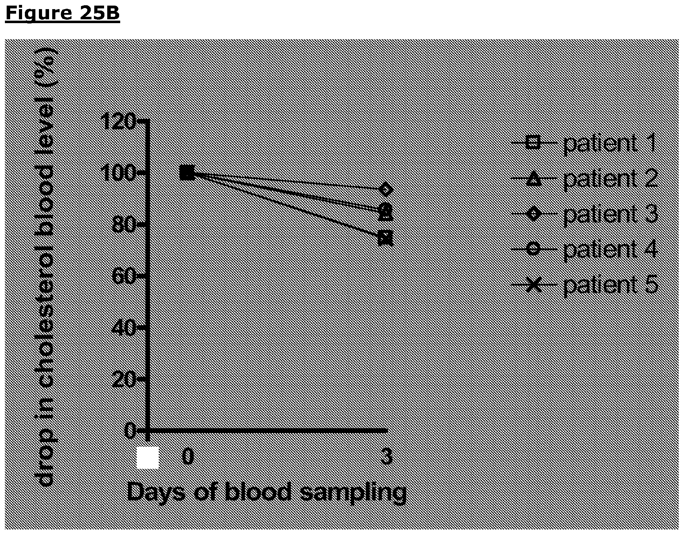

*Figure 25* shows the cholesterol blood concentration of hypercholesterolemic patients upon *in vivo* atorvastatin treatment. Five hypercholesterolemic patients were enrolled into an exploratory clinical trial. A patient is considered hypercholesterolemic when having a cholesterol blood level of 190 mg/ml (as indicated by dotted line in A). *Figure 25A* shows the cholesterol blood concentration. *Figure 25B* shows the percentage of reduction in cholesterol blood level as compared to day 0 (100%). At the time of recruitment, patient I.D.#4 presented with cholesterol blood levels > 200 mg/ml. For patient I.D.#5 hypercholesterolemia can be argued. In all patients a significant drop in cholesterol blood levels was observed on day 3 upon treatment with a single dose of 80 mg atorvastatin at day 0. The average cholesterol blood concentration of five patients diminished with 17% (± 4 SE) from 207 mg/ml on day 0 to 172 mg/ml on day 3 (A; P=0.0062, paired t-test, B. P=0.0082, paired t-test).

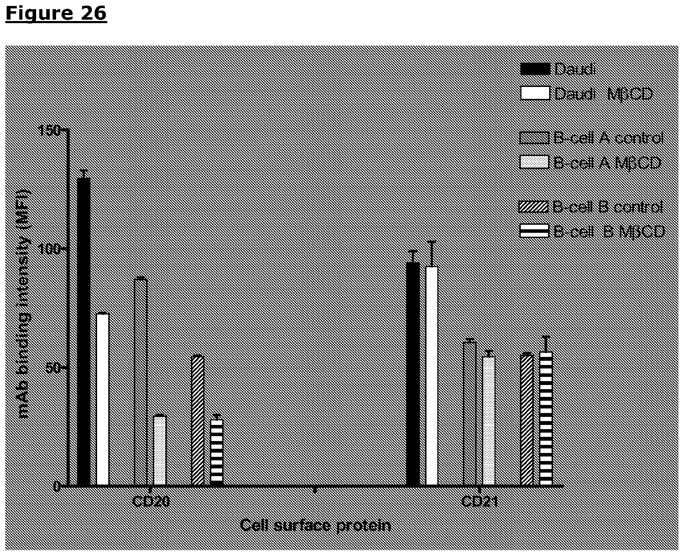

*Figure 26* shows anti-CD20 mAb and anti-CD21 mAb binding to cholesterol-depleted Daudi cells and freshly isolated B cell enriched populations. B cells were isolated by negative depletion out of a PBMC cell supsension using Dynabeads (according to manufacturer's instructions, Dynal Inc. InVitrogen, Carlsbad, CA). Daudi cells and freshly isolated B cells were incubated with MβCD (10 mg/ml) for 30 minutes, and subsequently washed and stained with FITC-conjugated CD20 mAb and PE-conjugated CD21 mAb. MAb binding was dectected using a flow cytometer.

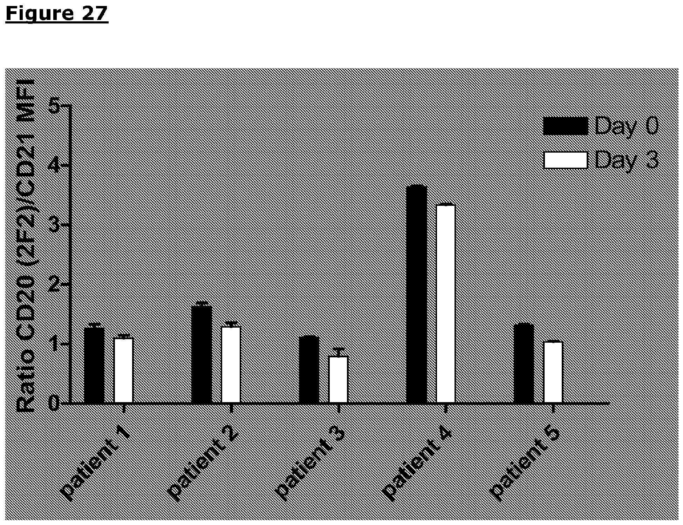

*Figure 27* shows that *in vivo* cholesterol depletion of hypercholesterolemic patients results in a decreased CD20 expression as detected using 2F2. Freshly isolated B cells of hypercholesterolemic patients treated with 80 mg atorvastatin were stained with FITC-conjugated 2F2 and PE-conjugated anti-CD21 mAb (B-Ly4) on day 0 and day 3, respectively. Anti-CD20 mAb binding intensity (MFI) was correlated to anti- CD21 mAb binding intensity (to correct for the amount of B cells analysed, and to be able to correlate results obtained in between days) by calculating the ratio of CD20/CD21 MFI.

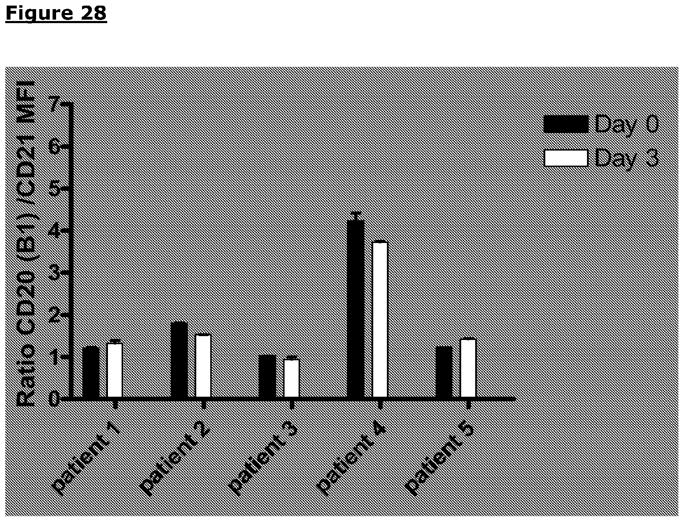

*Figure 28* shows that *in vivo* cholesterol depletion of hypercholesterolemic patients results in a decreased CD20 expression as detected using B1. Freshly isolated B cells of hypercholesterolemic patients treated with 80 mg atorvastatin were stained with FITC-conjugated CD20 B1 and PE-conjugated anti-CD21 mAb (B-Ly4) on day 0 and day 3, respectively. Anti-CD20 mAb binding intensity (MFI) was correlated to anti-CD21 mAb binding intensity (to correct for the amount of B cells analysed, and to be able to correlate results obtained in between days) by calculating the ratio of CD20/CD21 MFI.

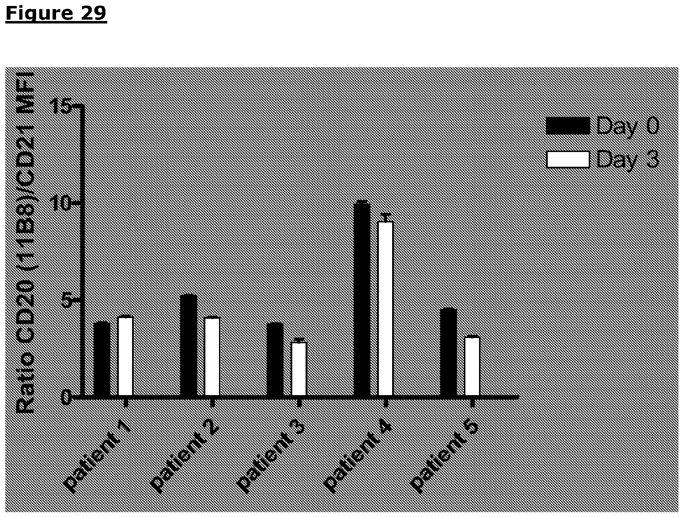

*Figure 29* shows that *in vivo* cholesterol depletion of hypercholesterolemic patients results in a decreased CD20 expression as detected using 11 B8. Freshly isolated B cells of hypercholesterolemic patients treated with 80 mg atorvastatin were stained with FITC-conjugated CD20 11 B8 and PE-conjugated anti-CD21 mAb (B-Ly4) on day 0 and day 3, respectively. Anti-CD20 mAb binding intensity (MFI) was correlated to anti-CD21 mAb binding intensity (to correct for the amount of B cells analysed, and to be able to correlate results obtained in between days) by calculating the ratio of CD20/CD21 MFI.

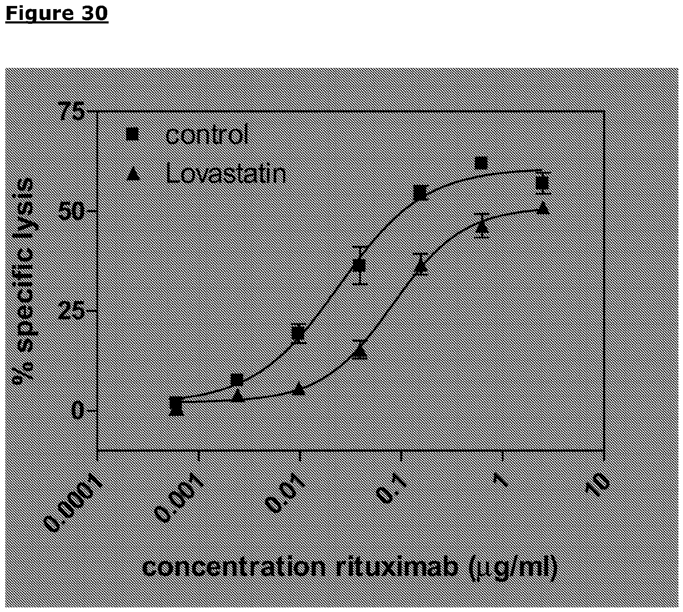

*Figure 30* shows that cholesterol depletion reduces ADCC-mediated lysis of RAJI cells in the presence of rituximab and PBMCs. Lovastatin-treated or control RAJI cells were used in a 51Cr-release assay to determine the ADCC-mediated lysis in the presence of a concentration curve of rituximab. Isolated PBMCs were used as effector cells.

## DETAILED DESCRIPTION OF THE INVENTION

The invention relates to an antibody capable of mediating CDC, which specifically binds to CD20, and which comprises a $V_H$ CDR1 of SEQ ID NO:3, a $V_H$ CDR2 of SEQ ID NO:4, a $V_H$ CDR3 of SEQ ID NO:5, a $V_L$ CDR1 of SEQ ID NO:6, a $V_L$ CDR2 of SEQ ID NO:7 and a $V_L$ CDR3 sequence of SEQ ID NO:8, for use in the treatment of a disease or disorder associated with said antigen, wherein the disease or disorder is selected from B cell lymphoma, B cell leukemia or an autoimmune disease, and wherein antibody-induced CDC has a beneficial effect on said disease or disorder, wherein the antibody is to be administered to a subject undergoing therapy with a cholesterol-lowering agent, such as a statin, and wherein the subject is withdrawn from treatment with the cholesterol-lowering agent prior to the administration of the antibody.

In one embodiment the subject is withdrawn from treatment with the cholesterol-lowering agent for a period of from at least 7 days, such as 14 days, 30 days, 45 days, 60 days or 90 days prior to the first administration of the antibody until at least 7 days, such as 14 days, 30 days, 45 days, 60 days or 90 days after the last administration of the antibody in a regimen.

In one embodiment of the invention, the antibody is a monoclonal antibody, such as a human monoclonal antibody. The antibody may be a full-length antibody, such as a full-length IgG1 antibody, or an antibody fragment retaining binding specificity to the antigen, such as a scFv or a UniBody® molecule (a monovalent antibody as disclosed in WO 2007/059782).

In one embodiment of the invention, the antibody medicament is suitable for intravenous, intraperitoneal, inhalation, intrabronchial, intraalveolar, intramuscular, subcutaneous or oral administration, such as intravenous injection or infusion.

In one embodiment of the invention, the antibody medicament is suitable for administration of the antibody in an amount of from 10-2000 mg.

In one embodiment thereof, the antibody against CD20 comprises a $V_H$ CDR1-CDR3 spanning sequence of SEQ ID NO:10.

In one embodiment thereof, the antibody against CD20 has human heavy chain and human light chain variable regions comprising the amino acid sequences as set forth in SEQ ID NO:1 and SEQ ID NO:2, respectively; or amino acid sequences which are at least 95% homologous, and more preferably at least 98%, or at least 99% homologous to the amino acid sequences as set forth in SEQ ID NO:1 and SEQ ID NO:2, respectively.

In one embodiment of the invention the anti-CD20 antibody is ofatumumab (2F2) disclosed in WO 2004/035607

In a further embodiment thereof, the antibody is 2F2(ScFvHL)$_4$-Fc, as decribed by Guo Y et al. (2008) Cancer Res, 68(7):2400-2408.

In one embodiment thereof, the antibody against CD20 comprises a heavy chain variable region amino acid sequence derived from a human $V_H$ DP-44/D3-10/JH6b germline sequence (SEQ ID NO:12) and a light chain variable region amino acid sequence derived from a human $V_L$ L6/JK4 (SEQ ID NO:13) germline sequence; or a heavy chain variable region amino acid sequence derived from a human $V_H$3-09/D4-11/JH6b germline sequence (SEQ ID NO:14) and a light chain variable region amino acid sequence derived from a human $V_L$ L6/JK5 germline sequence (SEQ ID NO:15), wherein the human antibody specifically binds to CD20.

In one embodiment of the invention, the treatment further comprises one or more further therapeutic agents selected from

*anti-inflammatory agents,* such as aspirin and other salicylates, Cox-2 inhibitors, such as rofecoxib (Vioxx) and celecoxib (Celebrex), a steroidal drug, or a NSAID (nonsteroidal anti-inflammatory drug), such as ibuprofen (Motrin, Advil), fenoprofen (Nalfon), naproxen (Naprosyn), sulindac (Clinoril), diclofenac (Voltaren), piroxicam (Feldene), ketoprofen (Orudis), diflunisal (Dolobid), nabumetone (Relafen), etodolac (Lodine), oxaprozin (Daypro), and indomethacin (Indocin); *immunosuppressive agents,* such as cyclosporine (Sandimmune, Neoral) and azathioprine (Imural);

*DMARDs,* such as methotrexate (Rheumatrex), hydroxychloroquine (Plaquenil), sulfasalazine (Asulfidine), pyrimidine synthesis inhibitors, *e.g.*, leflunomide (Arava), IL-1 receptor blocking agents, *e.g.*, anakinra (Kineret), and TNF-$\alpha$ blocking agents, *e.g.*, etanercept (Enbrel), infliximab (Remicade) and adalimumab; *chemotherapeutics,* such as doxorubicin (Adriamycin), cisplatin (Platinol), bleomycin (Blenoxane), carmustine (Gliadel), cyclophosphamide (Cytoxan, Procytox, Neosar), and chlorambucil (Leukeran).

In one embodiment of the invention, the treatment further comprises a combination with chlorambucil and prednisolone; cyclophosphamide and prednisolone; cyclophosphamide, vincristine, and prednisone; cyclophosphamide, vincristine, doxorubicin, and prednisone; fludarabine and anthracycline; or a combination with other common multi-drugs regimens for NHL, such as disclosed, *e.g.*, in Non-Hodgkin's Lymphomas: Making sense of Diagnosis, Treatment, and Options, Lorraine Johnston, 1999, O'Reilly and Associates, Inc.

In one embodiment of the invention, the treatment further comprises combination with one or more antibodies, targeting the same or different antigen(s).

In one embodiment of the invention, the treatment further comprises radiotherapy and/or autologous peripheral stem cell or bone marrow transplantation. Such treatment may further be combined with one or more therapeuticatic agents, such as those mentioned above.

In one embodiment of the invention, the antibody is capable of mediating ADCC, and the antibody-induced ADCC has a beneficial effect on the disease or disorder to be treated.

Further embodiments thereof comprise one or more of the above feature(s).

In one embodiment thereof, the B cell lymphoma, B cell leukemia or autoimmune disease is selected from follicular lymphoma (FL), chronic lymphocytic leukemia (CLL), diffuse large B-cell lymphoma (DLBCL), rheumatoid arthritis (RA), systemic lupus erythematosus (SLE), multiple sclerosis (MS), Sjögren's syndrome (SS), and chronic obstructive pulmonary disease (COPD).

In another embodiment thereof, the disease or disorder is Waldenström's macroglobulinemia.

In a particular embodiment, the disease or disorder is B cell lymphoma, *e.g.* non-Hodgkin's lymphoma (NHL). CD20 is usually expressed at elevated levels on neoplastic (*i.e.,* tumorigenic) B cells associated with NHL. Accordingly, the anti-CD20 antibody of the invention can be used to deplete CD20 bearing tumor cells which lead to NHL and, thus, can be used to prevent or treat this disease.

Non-Hodgkin's lymphoma is a type of B cell lymphoma. Lymphomas, *e.g.,* B cell lymphomas, are a group of related cancers that arise when a lymphocyte (a blood cell) becomes malignant. The normal function of lymphocytes is to defend the body against invaders: germs, viruses, fungi, even cancer. There are many subtypes and maturation stages of lymphocytes and, therefore, there are many kinds of lymphomas. Like normal cells, malignant lymphocytes can move to many parts of the body. Typically, lymphoma cells form tumors in the lymphatic system: bone marrow, lymph nodes, spleen, and blood. However, these cells can migrate to other organs. Certain types of lymphoma will tend to grow in locations in which the normal version of the cell resides. For example, it is common for follicular NHL tumors to develop in the lymph nodes.

Examples of non-Hodgkin's lymphoma (NHL) include precursor B cell lymphoblastic leukemia/lymphoma and mature B cell neoplasms, such as B cell chronic lymhocytic leukemia (CLL)/small lymphocytic lymphoma (SLL), B cell prolymphocytic leukemia, lymphoplasmacytic lymphoma, mantle cell lymphoma (MCL), follicular lymphoma (FL), including low-grade, intermediate-grade and high-grade FL, cutaneous follicle center lymphoma, marginal zone B cell lymphoma (MALT type, nodal and splenic type), hairy cell leukemia, diffuse large B-cell lymphoma (DLBCL), Burkitt's lymphoma, plasmacytoma, plasmacell myeloma, post-transplant lymphoproliferative disorder, Waldenström's macroglobulinemia, anaplastic large-cell lymphoma (ALCL), lymphomatoid granulomatosis, primary effusion lymphoma, intravascular large B cell lymphoma, mediastinal large B cell lymphoma, heavy chain diseases (including $\gamma$, $\mu$, and $\alpha$ disease), lymphomas induced by therapy with immunosuppressive agents, such as cyclosporine-induced lymphoma, and methotrexate-induced lymphoma.

In one embodiment the disease is follicular lymphoma (FL). In another embodiment the disease is lymhocytic leukemia (CLL)/small lymphocytic lymphoma (SLL). In yet another embodiment the disease is diffuse large B-cell lymphoma (DLBCL).

In a further embodiment, the anti-CD20 antibody of the present invention can be used to treat Hodgkin's lymphoma.

The anti-CD20 antibody (*e.g.*, human monoclonal antibody, multispecific and bispecific molecules) of the present invention also can be used to block or inhibit other effects of CD20. For example, it is known that CD20 is expressed on B lymphocytes and is involved in the proliferation and/or differentiation of these cells. Since B lymphocytes function as immunomodulators, CD20 is an important target for antibody mediated therapy to target B lymphocytes, *e.g.*, to inactivate or kill B lymphocytes, involved in immune, autoimmune, inflammatory or infectious disease or disorder involving human CD20 expressing cells.

Examples of diseases and disorders in which CD20 expressing B cells are involved and which can be treated and/or prevented include immune, autoimmune, inflammatory and infectious diseases and disorders, such as psoriasis, psoriatic arthritis, dermatitis, systemic sclerosis, inflammatory bowel disease (IBD), Crohn's disease, ulcerative colitis, respiratory distress syndrome, meningitis, encephalitis, uveitis, glomerulonephritis, eczema, asthma, atherosclerosis, leukocyte adhesion deficiency, multiple sclerosis (MS), Raynaud's syndrome, Sjögren's syndrome (SS), juvenile onset diabetes, Reiter's disease, Behçet's disease, immune complex nephritis, IgA nephropathy, IgM polyneuropathies, immune-mediated thrombocytopenias, such as acute idiopathic thrombocytopenic purpura and chronic idiopathic thrombocytopenic purpura, hemolytic anemia, myasthenia gravis, lupus nephritis, systemic lupus erythematosus (SLE), rheumatoid arthritis (RA), atopic dermatitis, pemphigus, Graves' disease, severe acute respiratory distress syndrome, choreoretinitis. Hashimoto's thyroiditis, Wegener's granulomatosis, Omenn's syndrome, chronic renal failure, acute infectious mononucleosis, HIV, herpes virus associated diseases, as well as diseases and disorders caused by or mediated by infection of B cells with virus, such as Epstein-Barr virus (EBV).

Further examples of inflammatory, immune and/or autoimmune disorders in which autoantibodies and/or excessive B lymphocyte activity are prominent and which can be treated and/or prevented, include the following:

*vasculitides and other vessel disorders,* such as microscopic polyangiitis, Churg-Strauss syndrome, and other ANCA-associated vasculitides, polyarteritis nodosa, essential cryoglobulinaemic vasculitis, cutaneous leukocytoclastic angiitis, Kawasaki disease, Takayasu arteritis, giant cell arthritis, Henoch-Schönlein purpura, primary or isolated cerebral angiitis, erythema nodosum, thrombangiitis obliterans, thrombotic thrombocytopenic purpura (including hemolytic uremic syndrome), and secondary vasculitides, including cutaneous leukocytoclastic vasculitis (*e.g.*, secondary to hepatitis B, hepatitis C, Waldenström's macroglobulinemia, B cell neoplasias, rheumatoid arthritis (RA), Sjögren's syndrome (SS), and systemic lupus erythematosus (SLE)), erythema nodosum, allergic vasculitis, panniculitis, Weber-Christian disease, purpura hyperglobulinaemica, and Buerger's disease;

*skin disorders,* such as contact dermatitis, linear IgA dermatosis, vitiligo, pyoderma gangrenosum, epidermolysis bullosa acquisita, pemphigus vulgaris (including cicatricial pemphigoid and bullous pemphigoid), alopecia areata (including alopecia universalis and alopecia totalis), dermatitis herpetiformis, erythema multiforme, and chronic autoimmune urticaria (including angioneurotic edema and urticarial vasculitis);

*immune-mediated cytopenias,* such as autoimmune neutropenia, and pure red cell aplasia;

*connective tissue disorders,* such as CNS lupus, discoid lupus erythematosus, CREST syndrome, mixed connective tissue disease, polymyositis/dermatomyositis, inclusion body myositis, secondary amyloidosis, cryoglobulinemia type I and type II, fibromyalgia, phospholipid antibody syndrome, secondary hemophilia, relapsing polychondritis, sarcoidosis, stiff man syndrome, rheumatic fever, and eosinophil fasciitis;

*arthritides,* such as ankylosing spondylitis, juvenile chronic arthritis, adult Still's disease, SAPHO syndrome, sacroileitis, reactive arthritis, Still's disease, and gout;

*hematologic disorders,* such as aplastic anemia, primary hemolytic anemia (including cold agglutinin syndrome), hemolytic anemia with warm autoantibodies, hemolytic anemia secondary to CLL or systemic lupus erythematosus (SLE); POEMS syndrome, pernicious anemia, Waldemström's purpura hyperglobulinaemica, Evans syndrome, agranulocytosis, autoimmune neutropenia, Franklin's disease, Seligmann's disease, μ-chain disease, factor VIII inhibitor formation, factor IX inhibitor formation, and paraneoplastic syndrome secondary to thymoma and lymphomas;

*endocrinopathies,* such as polyendocrinopathy, and Addison's disease; further examples are autoimmune hypoglycemia, autoimmune hypothyroidism, autoimmune insulin syndrome, de Quervain's thyroiditis, and insulin receptor antibody-mediated insulin resistance;

*hepato-gastrointestinal disorders,* such as celiac disease, Whipple's disease, primary biliary cirrhosis, chronic active hepatitis, primary sclerosing cholangiitis, and autoimmune gastritis;

*nephropathies,* such as rapid progressive glomerulonephritis, post-streptococcal nephritis, Goodpasture's syndrome, membranous glomerulonephritis, cryoglobulinemic nephritis, minimal change disease, and steroid-dependent nephritic syndrome;

*neurological disorders,* such as autoimmune neuropathies, mononeuritis multiplex, Lambert-Eaton's myasthenic syndrome, Sydenham's chorea, tabes dorsalis, and Guillain-Barré's syndrome; further examples are myelopathy/tropical spastic paraparesis, myasthenia gravis, acute inflammatory demyelinating polyneuropathy, and chronic inflammatory demyelinating polyneuropathy;

*cardiac and pulmonary disorders,* such as fibrosing alveolitis, bronchiolitis obliterans, allergic aspergillosis, cystic fibrosis, Löffler's syndrome, myocarditis, and pericarditis; further examples are hypersensitivity pneumonitis, and paraneoplastic syndrome secondary to lung cancer;

*allergic disorders,* such as bronchial asthma, hyper-IgE syndrome, and angioneurotic syndrome;

*ophthalmologic disorders,* such as idiopathic chorioretinitis, and amaurosis fugax;

*infectious diseases,* such as parvovirus B infection (including hands-and-socks syndrome);

*gynecological-obstretical disorders,* such as recurrent abortion, recurrent fetal loss, intrauterine growth retardation, and paraneoplastic syndrome secondary to gynaecological neoplasms;

*male reproductive disorders,* such as paraneoplastic syndrome secondary to testicular neoplasms; and

*transplantation-derived disorders,* such as allograft and xenograft rejection, and graft-versus-host disease.

In one embodiment, the disease is rheumatoid arthritis (RA).

In another embodiment, the disease is selected from inflammatory bowel disease (IBD), ulcerative colitis, Crohn's disease, juvenile onset diabetes, multiple sclerosis (MS), immune-mediated thrombocytopenias, such as acute idiopathic thrombocytopenic purpura and chronic idiopathic thrombocytopenic purpura, hemolytic anemia (including autoimmune hemolytic anemia), myasthenia gravis, systemic sclerosis, and pemphigus vulgaris.

In yet a further embodiment of the invention, the disease is selected from rheumatoid arthritis (RA), systemic lupus erythematosus (SLE), multiple sclerosis (MS), Sjögren's syndrome (SS), and chronic obstructive pulmonary disease (COPD).

In yet a further embodiment of the invention, the disease is Waldenström's macroglobulinemia.

The antibody may be administered via any suitable route, such as an oral, nasal, inhalable, intrabronchial, intraalveolar, topical (including buccal, transdermal and sublingual), rectal, vaginal and/or parenteral route

In one embodiment, the antibody composition is administered parenterally.

The phrases "parenteral administration" and "administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In one embodiment, the antibody composition is administered by intravenous or subcutaneous injection or infusion. For example the pharmaceutical composition may be administered over 2-8 hours, such as 4 hours, in order to reduce side effects.

In one embodiment, the pharmaceutical composition is administered by inhalation. Fab fragments of antibodies may be suitable for such administration route, cf. Crowe et al. (February 15, 1994) Proc Natl Acad Sci USA, 91(4):1386-1390.

In one embodiment, the pharmaceutical composition is administered in crystalline form by subcutaneous injection, cf. Yang et al., PNAS USA 100(12), 6934-6939 (2003).

Regardless of the route of administration selected, the antibody, which may be used in the form of a pharmaceutically acceptable salt or in a suitable hydrated form, are formulated into pharmaceutically acceptable dosage forms by conventional methods known to those of skill in the art. A "pharmaceutically acceptable salt" refers to a salt that retains the desired biological activity of the parent compound and does not impart any undesired toxicological effects (see for instance Berge, S.M. et al., J. Pharm. Sci. 66, 1-19 (1977)). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous acids and the like, as well as from nontoxic organic acids, such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

Pharmaceutically acceptable carriers include any and all suitable solvents, dispersion media, coatings, antibacterial and antifungal agents, isotonicity agents, antioxidants and absorption delaying agents, and the like that are physiologically compatible with a compound of the present invention.

Examples of suitable aqueous and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present invention is contem plated.

Proper fluidity may be maintained, for example, by the use of coating materials, such as lecithin, by the maintenance of the required particle size in the case of dispersions, and by the use of surfactants.

Pharmaceutical compositions containing the antibody may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Pharmaceutical compositions of the present invention may also comprise isotonicity agents, such as sugars, polyalcohols such as mannitol, sorbitol, glycerol or sodium chloride in the compositions.

Pharmaceutically acceptable diluents include saline and aqueous buffer solutions.

The pharmaceutical compositions containing the antibody may also contain one or more adjuvants appropriate for the chosen route of administration, such as preservatives, wetting agents, emulsifying agents, dispersing agents, preservatives or buffers, which may enhance the shelf life or effectiveness of the pharmaceutical composition. Compounds of the present invention may for instance be admixed with lactose, sucrose, powders (e.g., starch powder), cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol. Other examples of adjuvants are QS21, GM-CSF, SRL-172, histamine dihydrochloride, thymocartin, Tio-TEPA, monophosphoryl-lipid A/microbacteria compositions, alum, incomplete Freund's adjuvant, montanide ISA, ribi adjuvant system, TiterMax adjuvant, syntex adjuvant formulations, immune-stimulating complexes (ISCOMs), gerbu adjuvant, CpG oligodeoxynucleotides, lipopolysaccharide, and polyinosinic:polycytidylic acid.

Prevention of presence of microorganisms may be ensured both by sterilization procedures and by the inclusion of various antibacterial and antifungal agents, for example, paraben, chlorobutanol, phenol, sorbic acid, and the like. In addition, prolonged absorption of the injectable pharmaceutical form may be brought about by the inclusion of agents which delay absorption, such as aluminum monostearate and gelatin.

The pharmaceutical compositions containing the antibody comprising a compound of the present invention may also include a suitable salt therefore. Any suitable salt, such as an alkaline earth metal salt in any suitable form (e.g., a buffer salt), may be used in the stabilization of the compound of the present invention. Suitable salts typically include sodium chloride, sodium succinate, sodium sulfate, potassium chloride, magnesium chloride, magnesium sulfate, and calcium chloride. In one embodiment, an aluminum salt is used to stabilize a compound of the present invention in a pharmaceutical composition of the present invention, which aluminum salt also may serve as an adjuvant when such a composition is administered to a patient.

The pharmaceutical compositions containing the antibody may be in a variety of suitable forms. Such forms include, for example, liquid, semi-solid and solid dosage forms, such as liquid solutions (e.g., injectable and infusible solutions), dispersions or suspensions, emulsions, microemulsions, gels, creams, granules, powders, tablets, pills, powders, liposomes, dendrimers and other nanoparticles (see for instance Baek et al., Methods Enzymol. 362, 240-9 (2003), Nigavekar et al., Pharm Res. 21(3), 476-83 (2004), microparticles, and suppositories.

The optimal form depends on the mode of administration chosen and the nature of the composition. Formulations may include, for instance, powders, pastes, ointments, jellies, waxes, oils, lipids, lipid (cationic or anionic) containing vesicles, DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing may be appropriate in treatments and therapies in accordance with the present invention, provided that the antibody in the pharmaceutical composition is not inactivated by the formulation and the formulation is physiologically compatible and tolerable with the route of administration. See also for instance Powell et al., "Compendium of excipients for parenteral formulations" PDA J Pharm Sci Technol. 52, 238-311 (1998) and the citations therein for additional information related to excipients and carriers well known to pharmaceutical chemists.

The anti-CD20 antibody may be prepared with carriers that will protect the compound against rapid release, such as a controlled release formulation, including implants, transdermal patches, and microencapsulated delivery systems. Such carriers may include gelatin, glyceryl monostearate, glyceryl distearate, biodegradable, biocompatible polymers, such as ethylene vinyl acetate, polyanhydrides, polyglycolic acid, collagen, polyorthoesters, and polylactic acid alone or with a wax, or other materials well known in the art.. Methods for the preparation of such formulations are generally known to those skilled in the art. See e.g., Sustained and Controlled Release Drug Delivery Systems, J.R. Robinson, ed., Marcel Dekker, Inc., New York, 1978.

To administer the pharmaceutical compositions containing the antibody by certain routes of administration according to the invention, it may be necessary to coat the antibody with, or co-administer the antibody with, a material to prevent its inactivation. For example, the antibody may be administered to a subject in an appropriate carrier, for example, liposomes, or a diluent. Liposomes include water-in-oil-in-water CGF emulsions as well as conventional liposomes (Strejan et al., J. Neuroimmunol. 7, 27 (1984)).

Depending on the route of administration, the antibody may be coated in a material to protect the antibody from the action of acids and other natural conditions that may inactivate the compound. For example, the antibody may be administered to a subject in an appropriate carrier, for example, liposomes. Liposomes include water-in-oil-in-water CGF emulsions as well as conventional liposomes (Strejan et al., J. Neuroimmunol. 7, 27 (1984)).

Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present invention is contemplated. Supplementary active compounds may also be incorporated into the compositions.

Pharmaceutical compositions for injection must typically be sterile and stable under the conditions of manufacture and storage. The composition may be formulated as a solution, microemulsion, liposome, or other ordered structure suitable to high drug concentration. The carrier may be a aqueous or nonaqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating, such as lecithin, by the maintenance of the required particle size in the case of dispersion and by the use of surfactants. In many cases, it will be preferable to include isotonic agents, for example, sugars, polyalcohols, such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the active compound in the required amount in an appropriate solvent with one or a combination of ingredients e.g. as enumerated above, as required, followed by sterilization microfiltration.

Generally, dispersions are prepared by incorporating the active compound into a sterile vehicle that contains a basic dispersion medium and the required other ingredients e.g. from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

Sterile injectable solutions may be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

The pharmaceutical composition may contain a combination of multiple (e.g., two or more) antibodies targeting the same antigen which act by different mechanisms, e.g., one anti-CD20 antibody which predominately acts by inducing CDC in combination with another anti-CD20 antibody which predominately acts by inducing apoptosis.

## EXAMPLES

The present invention is further illustrated by the following examples which should not be construed as further limiting.

### Example 1 : Culture and characterization of cell lines used for the experiment

The following CD20 expressing cell lines were used in the experiments:

Daudi: a human negroid Burkitt's lymphoma cell line obtained from ECACC, Porton Down, United Kingdom

RAJI and RAJI-CD20high: human negroid Burkitt's lymphoma cell lines obtained from ECACC, Porton Down, United Kingdom. The two RAJI cell lines differ in binding intensity of anti-CD20 mAbs. Furtherhmore, RAJI-CD20high cell line shows a higher binding of mAbs directed against all other cell surface proteins except for the complement regulatory protein CD59 and the B cell protein CD19 which showed a lower expression compared to RAJI cells. Both cell lines did not stain positive for CD3 (negative control).

These cell lines were cultured in RPMI 1640 supplemented with 10% heat-inactivated cosmic calf serum (CCS), 1 U/ml penicillin, 1 $\mu$g/ml streptomycin, and 4 mM L-glutamine (all from Invitrogen, Carlsbad, CA).

WIL2-S: A hereditary spherocytosis cell line WIL2-S (ATCC, Teddington, United Kingdom) was cultured in HyQADCF (Hyclone, Logan, Utah, United states) supplemented with 100 U/ml penicillin, 100 U/ml streptomycin and 100 mM sodium pyruvate (all from Invitrogen, Carlsbad, CA).

For the functional experiments, viability of the cell lines was tested by 0.4% trypan blue (Sigma, Zwijndrecht, Netherlands) exclusion. The cell lines were washed twice in PBS and re-suspended in test medium (RPMI 1640, 100 U/ml penicillin, 100 U/ml streptomycin, 0.1% BSA) at a concentration of $2x10^6$ viable cells/ml.

### Example 2: Depletion of cholesterol from membranes of various CD20 expressing B cell lines using methyl-beta-cyclodextrin (MβCD)

To deplete cholesterol from the cell membrane, methyl-beta-cyclodextrin (MβCD, Sigma, Zwijndrecht, The Netherlands) was added in varying concentrations to Daudi cells and RAJI-CD20high cells and incubated for 30 minutes at 37°C under gentle shaking conditions. After incubation the cells were washed twice in PBS and resuspended in test medium to a concentration of $2x10^6$ viable cells/ml. MβCD-treated Daudi cells ($1x10^5$) were incubated with a saturating concentration of FITC-conjugated human anti-CD20 mAb 2F2 or the anti-CD20 mAb B9E9 (Beckman Coulter Inc., Fullerton, CA) for 30 minutes at 4°C. After washing twice with FACS buffer (PBS, 0.1% Bovine Serum Albumine, 0.02% Sodium Azide), cells were analyzed on a FACS Calibur (Becton Dickinson, Breda, The Netherlands). A dose-dependent decrease in binding of FITC-conjugated anti-CD20 mAb 2F2 to CD20 expressed by MβCD-treated Daudi cells was observed (Figure 1 A). Binding is expressed as mean fluorescence intensity. Also in RAJI-CD20high cells incubated with 5 mg/ml MβCD a decreased binding of FITC-conjugated B9E9 as compared to non-treated cells was observed using flow cytometry (Figure 1 B).

### Example 3: Depletion of cholesterol from membranes of various CD20 expressing B cell lines using statins diminishes anti-mAb induced CDC

Statins are competitive inhibitors of 3-hydroxy-3-methylglutaryl-coenzyme A reductase (HMG-CoAR). HMG-CoAR is a rate-limiting enzyme of the mevalonate pathway essential for the synthesis of isoprenoid compounds including cholesterol (McTaggart SJ (2006) Isoprenylated proteins. Cell Mol Life Sci 63:255-267). By inhibiting HMG-CoAR, statins can lower the cholesterol blood level and extract cholesterol from the cell membrane. To determine the effect of statin-mediated cholesterol depletion on immunotherapy RAJI-CD20high cells were cultured with a concentration range of lovastatin or diluent for 48 hours.

Lovastatin-treated cells were subjected to mAb-induced CDC by incubating cells ($1x10^5$/well) for 60 minutes with 10 μg/ml rituximab in the presence of 10% complement active serum. Cell viability was measured in a MTT assay in which 3-(4,5-dimethylthiazol-2-yl)-2,5-diphenyltetrazolium bromide (MTT) solution (5 mg/ml, Roche, Diagnostics, Almere, The Netherlands) was added to each well (flat-bottom microtiter plates; Nunc, Rochester, NY). The reaction was stopped after 4 hours of incubation at 37°C by the addition of 100 μl of 20% SDS (Roche Diagnostics, Almere, The Netherlands). After overnight incubation at 37°C the absorbance of the samples was measured at 550-525 nm using an Elisa reader (EL808x, Biotek instruments, Highland park, Vermont, USA). Cytotoxicity was expressed as a relative viability of the rituximab-mediated CDC of lovastatin-treated cells compared to cells incubated with medium only and was calculated as follows: relative viability =[(Ae-Ab)/(Ac-Ab)]* 100, where Ab is the background absorbance, Ae is experimental absorbance of the lysed cells and Ac is the absorbance of untreated cells. The viability was expressed as percentage survival in comparison to non-cholesterol depleted cells (control).

A concentration-dependent protection of RAJI-CD20high cells from rituximab-induced CDC was observed upon incubation of cells with lovastatin for 48 hours (Figure 2). Thus, depletion of cholesterol by statins diminished the complement-mediated lysis induced in the presence of rituximab and complement.

### Example 4: Reconsitution of cholesterol into membranes of CD20 expressing B cell line after depletion of cholesterol reinstates binding of anti-CD20 mAb

Cholesterol was depleted from RAJI-CD20high cell line using the cholesterol-depleting agents MβCD or lovastatin, as described in Examples 2 and 3, respectively. The cells were washed twice in PBS and resuspended in test medium to a concentration of $2x10^6$ viable cells/ml. As a control an untreated RAJI-CD20high cell line was included.

To reconstitute cholesterol into the cell membrane of the cells or increase the cholesterol content of the untreated cells, cholesterol (ICN Biomedicals, Zoetermeer, The Netherlands) was added to the test medium in a final concentration of 5 mg/ml 2 hours prior to FACS analysis. The cells were incubated for 30 minutes at 37°C under gentle shaking conditions, after which the cells were washed twice in PBS and resuspended to $2x10^6$ viable cells/ml. For comparison MβCD- and lovastatin-treated cell lines treated with only diluent were included.

To determine the effect of adding cholesterol on CD20 mAb binding, all cell lines ($1x10^5$) were incubated with

a saturating concentration of B9E9 for 30 minutes at 4°C. After washing twice with FACS buffer (PBS, 0.1% Bovine Serum Albumine, 0.02% Sodium Azide), the cells were analyzed on a FACS Calibur (Becton Dickinson, Breda, The Netherlands).

Incubation of RAJI-CD20high cells with cholesterol upon pre-treatment with either lovastatin (Figure 3A) or MβCD (Figure 3B) completely reinstated binding of B9E9, indicating that cholesterol is necessary for CD20 binding.

**Example 5: Expression of cell surface proteins on various B cell lines after depletion or reconstitution of cholesterol**

B cells (either non-treated cells, cholesterol-depleted cells via MβCD or lovastatin, or cholesterol-reconstituted cells) were characterized for quantitative surface expression of the B cell markers CD19, CD20, and CD21, the T-cell marker CD3, complement regulatory proteins CD55 and CD59, and HLA-DR and CD81 by direct immunofluorescence. $1 \times 10^5$ target cells were incubated with human anti-CD20 mAb 2F2 as well as mouse mAb SJ25CI (CD19-PE), B-Ly4 (CD21-PE), UCHT-1 (CD3-APC), IA10 (CD55-APC), p282 (CD59-FITC,) TU36 (HLA-DR-PE), and JS-81 (CD81-PE) (all from BD Pharmingen, Franklin Lakes, NJ) at saturating concentrations for 30 minutes at 4°C. After washing twice with FACS buffer (PBS, 0.1% Bovine Serum Albumine, 0.02% Sodium Azide), the cells were analyzed on a flow cytometer.

Binding of various mAbs to RAJI cells incubated with increasing concentrations of MβCD as cholesterol depleting agent and measured by FACS analysis is shown in Figure 4A and Figure 4B. Binding of mAbs directed against CD19, CD20, CD55, CD59, HLA-DR and CD81 is negatively correlated to the concentration of MβCD such that mAb binding is diminished when the MβCD concentration is increased. However, mAb binding to CD21 is not affected by an increased MβCD concentration (P>0.05, Kruskal Wallis test). Anti-CD3 mAb binding served as negative control.

Similar observations were made when depleting cholesterol from the membrane upon culture of RAJI cells with 10 μM of lovastatin (Figure 5A and Figure 5B). Lovastatin-mediated cholesterol depletion resulted in a clearly decreased mAb binding of CD19 and CD20, but not of the B cell surface protein CD21 (P>0.05, t-test). Although CD59 showed a slightly reduced mAb staining, binding of mAb to the complement regulatory protein was not affected by lovastatin. The same was observed for HLA-DR and CD81. Anti-CD3 mAb binding served as negative control.

Binding of various mAbs to cell surface proteins of WIL2-S cells replenished with cholesterol after incubation with lovastatin showed that binding of most mAb is dependent on the cholesterol concentration in the cell membrane (Figure 6A and Figure 6B). As CD21 is not expressed and CD19 and CD59 are hardly expressed by WIL2-S cells no conclusions could be drawn with regard to the effect of changing the cholesterol membrane content on mAb binding to these proteins. Anti-CD20 mAb and anti-HLA-DR mAb binding was improved upon addition of extra cholesterol into the cell culture compared to non-treated cells (dark grey bars). Depletion of cholesterol via lovastatin incubation of WIL2-S cells diminished anti-CD20 and anti-HLA mAb binding (black bars) compared to non-treated cells (white bars), as also observed for RAJI and Daudi cells. This could be restored upon reconstitution of cholesterol of lovastatin-treated cells (dark grey bars). Anti-CD3 mAb binding served as negative control.

**Example 6: Binding of anti-CD20 mAbs to CD20 on MβCD-mediated cholesterol-depleted B cells**

Cholesterol was depleted from the cell membrane of various B cells using varying concentrations of MβCD (as described in Example 2). Cells ($1 \times 10^5$ cells/staining) were incubated with a serial dilution of human CD20 mAb 2F2 starting at saturating concentrations for 30 minutes at 4°C. After washing with FACS buffer, polyclonal rabbit-anti-human IgG-FITC (DAKO A/S, Denmark) was added for 30 minutes at 4°C. The cells were washed again, resuspended in FACS buffer and analyzed by flow cytometry. The mean fluorescence intensity (MFI) is a measure for CD20 binding of the mAb. Sigmoidal dose-response curves were calculated using non-linear regression and the $EC_{50}$ of mAb binding were determined (using GraphPad Prism 4 statistical software).

A decrease in anti-CD20 mAb binding on Daudi cells incubated with a dose range of MβCD was observed (Figure 1). To determine whether this might be due to a reduced affinity for binding of the mAb a concentration curve of CD20 mAb binding was performed. Upon depletion of cholesterol using increasing concentrations of MβCD the binding affinity of 2F2 for CD20 was reduced ~ 8-fold as shown by the increase in $EC_{50}$ comparing 0 mg/ml vs 5 mg/ml MβCD (Figure 7A). The binding affinity of rituximab was also negatively affected when depleting cholesterol of Daudi cells (Figure 7B). Likewise the CD20 mAb 11B8 showed a reduced binding affinity upon MβCD-treatment of Daudi cells (Figure 7C). A similar (dose-dependent) decrease in binding affinity for CD20 mAbs was observed when treating RAJI cells with MβCD (data not shown).

**Example 7: Binding of anti-CD20 monoclonal antibodies to CD20 on statin-mediated cholesterol-depleted B cells**

Cholesterol was depleted from the cell membrane of various B cells using varying concentrations of lovastatin (as described in Example 3) and CD20 mAb binding was determined. Cells ($1 \times 10^5$ cells/staining) were incubated with a FITC-conjugated B9E9 for 30 minutes at 4°C. Cells were washed and resuspended in FACS buffer and analyzed by flow cytometry. The mean fluorescence intensity (MFI) is a measure for CD20 binding of the mAb.

Binding of B9E9 to CD20 was reduced on lovastatin-treated RAJI-CD20high cells compared to non-treated cells. RAJI-CD20 high cells were incubated with either diluent (control; black line) or lovastatin (10 $\mu$M for 48 hours; dark grey line). Treated cells were incubated with saturating amounts of FITC-conjugated B9E9 for 30 minutes at room temperature and analyzed using a flow cytometer. On the Y-axis the number of positive stained cells indicated by counts is shown, and on the X-axis the staining intensity is shown. Whereas over 95% of control RAJI-CD20high cells stained positive for B9E9, only 30% of cells showed significant binding to CD20 after 48 hours of treatment with 10 $\mu$M lovastatin (Figure 8).

To exclude the possibility that the presence of drugs might interfere with mAb binding the expression of HMG-CoAR was silenced with siRNA. 24 hours before transfection RAJI-CD20high cells were seeded from single-cell suspension at 2x $10^5$ cells/well in a 24 well plate. After overnight culture, cells were transfected with siRNA against HMG-CoAR (sequences provided by Qiagen) using HiPerFect Transfection Reagent (Qiagen) according to manufacturer's protocol. 24 hours after transfection cells were stained with anti-CD20 mAb to detect the expression of CD20, as described above. Inhibition of HMG-CoAR expression resulted in a decreased binding of anti-CD20 mAb to RAJI-CD20high cells (Figure 9).

This decrease in binding might be related to a decreased CD20 expression or a reduced mAb binding affinity, and this was studied in more detail in Examples 8 to 10.

**Example 8: Statins do not change the expression level of CD20 mRNA and protein**

In addition to lowering of cholesterol synthesis, statins exert pleitropic effects thereby influencing the expression of a number of genes, cf. Liao JK, Laufs U (2005) Pleiotropic effects of statins. Annu Rev Pharmacol Toxicol 45: 89-118; Alegret M, Silvestre JS (2006) Pleiotropic effects of statins and related pharmacological experimental approaches. Methods Find Exp Clin Pharmacol 28: 627-656; Ito MK, Talbert RL, Tsimikas S (2006) Statin-associated pleiotropy: possible effects beyond cholesterol reduction. Pharmacotherapy 26: 85S-97S.

To analyze whether lovastatin treatment would influence CD20 expression mRNA expression of the target was determined. RAJI-CD20high cells were incubated with either diluent (control) or 10 $\mu$M of lovastatin for different time periods (1 - 48 hours). Cells were washed twice with PBS, pelleted and treated with 1 ml of TRIzol Reagent (Invitrogen) to extract total RNA according to the manufacturer's protocol. RNA concentration was measured with Eppendorf Biophotometer (Eppendorf, Hamburg, Germany). The first strand cDNA synthesis containing 1 $\mu$g of total RNA was primed with oligo(dT) using Omniscript RT Kit (Qiagen, Chatsworth, CA). Primers used for CD20 PCR amplification were: forward - 5' TGAATGGGCTCTTCCACATTGCC3' and reverse - 5' CCTGGAAGAAGGCAAAGATCAGC3'. The cycling conditions in the Mastercycler personal (Eppendorf) consisted of a first step of 94°C denaturation for 10 minutes, followed by 35 cycles of annealing at 54°C for 60 sec, extension at 75°C for 90 sec, and denaturation at 94°C for 30 sec, with a final elongation step at 75°C for 10 minutes using HotStart Taq DNA Polymerase (Qiagen). Amplification products were analyzed by 1.5% agarose gel electrophoresis. The mRNA level for CD20 remained constant after lovastatin treatment in a time course over 48 hours (Figure 10, right panel).

Although CD20 mRNA expression remained constant, lovastatin might have affected the CD20 protein levels. Control cells or cells incubated with 10 $\mu$M lovastatin for 48 hours, washed twice with PBS, were pelleted and lysed with radioimmunoprecipitation assay (RIPA) buffer containing Tris base 50 mM, NaCl 150 mM, NP-40 1%, sodium deoxycholate 0.25% and EDTA 1 mM supplemented with Complete® protease inhibitor cocktail tablets (Roche Diagnostics, Mannheim, Germany). Protein concentration was measured using Bio-Rad Protein Assay (BioRad, Hercules, CA, USA). Equal amounts of whole cell proteins were separated on 12.5% SDS-polyacrylamide gel, transferred onto Protran® nitrocellulose membranes (Schleicher and Schuell BioScience Inc., Keene, NH), blocked with TBST (Tris buffered saline (pH 7.4) and 0.05% Tween 20) supplemented with 5% nonfat milk and 5% FBS. The following mAb (at 1:1000 dilution) were used for the overnight incubation: anti-CD20 (NCL-CD20-L26, Novocastra Laboratories Ltd, UK), anti-ICAM-1 (Santa Cruz, Santa Cruz, CA). After extensive washing with TBST, the membranes were incubated for 45 minutes with peroxidase conjugated ImmunoPure Goat Anti-Mouse IgG [F(ab')$_2$] (Jackson ImmunoResearch Laboratories Inc, PA). The chemiluminescence reaction for horseradish peroxidase was developed using SuperSignal WestPico Trail Kit® (Pierce, Rockford, IL) on a standard x-ray film. The blots were stripped in 0.1 M glycine pH 2.6 and reprobed with anti-tubulin mouse mAb (Calbiochem) to control for loading differences. 48 hours incubation with different concentrations of lovastatin (from 5-30 $\mu$M) did not influence the total cellular CD20 protein level (Figure 10, right panel).

**Example 9: CD20 is expressed on the plasma membrane of RAJI-CD20high cells upon lovastatin treatment**

Decreased binding of anti-CD20 molecules with simultaneous stable levels of m RNA and protein in total cell lysates might be explained by either retention/redistribution of CD20 in cytosolic compartments or shedding from the plasma membrane. To examine localization of CD20 in RAJI-CD20high cells a double immunofluorescence staining with anti-CD20 mAb directed against an extracellular conformational epitope of CD20, and, after cell permeabilization, with a mAb against a linear epitope in the cytosolic tail of this molecule was performed.

Control and lovastatin-pretreated RAJI-CD20high cells were stained in suspension at a density of $5 \times 10^5$/ml with FITC-conjugated B9E9 (1:10 in PBS, Immunotech Coulter Company, France) for 30 minutes at room temperature. After washing in PBS (three times), 200 µl of the cell suspension was spun onto a Cytospin slide. The slides were air-dried, acetone-fixed for 15 minutes at room temperature, washed three times with PBS, incubated with anti-CD20 mAb (Novocastra) (1:100 in PBS with 5% normal donkey serum (Jackson)) for 60 minutes at room temperature. The slides were washed three times in PBS and incubated with donkey anti-mouse Alexa555 conjugated antibody (Molecular Probes, CA) (1:200 for 30 minutes at room temperature). The slides were washed, mounted in Vectashield (Vector Laboratories, CA) and examined by fluorescence microscopy (Leica TCS SP2).

Figure 11 (A and B) shows the results of confocal experiments. While in untreated controls both mAb detected CD20 expressed on RAJI-CD20high cells, a 48-hour pre-treatment with lovastatin nearly completely abrogated binding of mAb directed to the extracellular epitope. However, mAb directed against the cytosolic tail of CD20 stained an antigen associated in the membrane. These experiments indicate that in lovastatin-treated cells CD20 is still present in the plasma membrane.

To further elucidate these observations control and lovastatin-treated cells were labeled with EZ-link® sulfo-NHS-biotin. This water-soluble and membrane impermeable reagent stably binds to primary amino (-$NH_2$) groups of extracellular portions of transmembrane proteins. Control and lovastatin-treated cells, washed three times with ice-cold PBS (pH 8.0) and resuspended at a density of 25x $10^6$ cells/ml were surface-labeled with 2 mM (final concentration) EZ-link® sulfo-NHS-biotin (Pierce) for 30 minutes at room temperature. Cells were washed three times (in PBS with 100 mM glycine) and lysed with RIPA lysis buffer containing proteases inhibitors (as described earlier, see Example 8). Biotinylated proteins were precipitated with immobilized NeutrAvidin protein (Pierce) by mixing the suspension for 1 hour at room temperature to separate the biotinylated surface protein from non-biotinylated ones. After five washes, gel-bound complexes were boiled in $2 \times$ Laemmli sample buffer and analyzed for CD20 by Western blotting using anti-CD20 mAb (NCL-CD20-L26, Novocastra) as described in Example 8.

Electrophoresis of the precipitates followed by blotting with anti-CD20 mAb (targeting the cytoplasmic portion of CD20) revealed that CD20 is detectable in lovastatin-treated cells in comparable amounts as compared with controls (Figure 11C). The expression of ICAM-1, another transmembrane protein was identical in control and lovastatin-treated cells. Together, these studies confirm that CD20 is present in the plasma membrane of lovastatin-treated cells.

### Example 10: Statins influence binding affinity of mAb directed to CD20

To confirm that lovastatin treatment might result in a change in CD20 mAb binding affinity lovastatin-treated RAJI cells were stained with a concentration curve of CD20 mAb. Cells ($1 \times 10^5$ cells/staining) were incubated with a serial dilution of CD20 mAb (2F2, rituximab or 11B8) starting at saturating concentrations for 30 minutes at 4°C. After washing with FACS buffer, polyclonal rabbit-anti-human IgG-FITC (DAKO A/S, Denmark) was added for 30 minutes at 4°C to detect the bound CD20 mAb. Cells were washed again, resuspended in FACS buffer and analyzed on a flow cytometer. The mean fluorescence intensity (MFI) was taken as measure for CD20 binding of the mAb.

The data confirmed that the lovastatin treatment resulted in a decrease in affinity of anti-CD20 mAb binding to the target as observed by the shift in the binding curves when compared to non-treated cells (control) (Figure 12).

Summarizing, the studies confirm that CD20 is present in the plasma membrane of lovastatin-treated cells, but that it becomes more difficult to target CD20 by binding of mAb directed against an extracellular epitope on the target.

### Example 11 : Binding of anti-CD20 monoclonal antibodies to CD20 expressed by cholesterol-treated B cells

Binding of anti-CD20 mAb to CD20 on various B cell lines could be restored upon replenishment of the cell membrane cholesterol content via addition of cholesterol (according to procedure as described in Example 4) to either MβCD- or lovastatin-treated cells, as measured by FACS analysis (Figure 3). This was also observed for WIL2-S cells for which binding affinity of anti-CD20 mAbs to CD20 was determined after reconstitution of cholesterol of cholesterol-depleted cells using flow cytometry (Figure 13). Moreover, addition of cholesterol to non-treated control cells further improved the binding affinity of both 2F2 (A), rituximab (B) and 11 B8 (C).

Data show a reduced affinity in binding of 2F2 (A), rituximab (B) or 11B8 (C) to cells depleted for cholesterol with lovastatin. Replenishing of cholesterol restores affinity to the level of non-treated cells.

In summary, depleting cholesterol from the cell membrane with either MβCD or lovastatin reduces the binding affinity of anti-CD20 mAb to CD20. Replenishment of the cholesterol membrane content restores or further improves the binding of the anti-CD20 mAb to CD20. This is observed for both 2F2, rituximab as well as 11B8.

**Example 12: Sensitivity to CD20 mAb-induced CDC of cholesterol-depleted Daudi cells detected via PI/ FACS analysis**

Cells were either non-treated or cholesterol-depleted as described in Example 2, Example 3 and Example 4, and resuspended in test medium to a concentration of $2 \times 10^6$ viable cells/ml. Daudi cells were used as target cells in a mAb-induced CDC assay. 50 μl of cell suspension was incubated with 50 μl anti-CD20 mAb in a serial dilution for 15 minutes at room temperature. After the incubation period, Normal Human Serum (M0008, Sanquin, Amsterdam, The Netherlands) was added as a source of complement (final concentration, 15%) to the cell suspension in round-bottomed microtiter plates (Nunc, Rochester, NY). The mixture was incubated for 45 minutes at 37°C after which the reaction was stopped by placing the samples on ice. Propidium iodide (PI, Sigma Aldrich, Zwijndrecht, The Netherlands) was added as a means to visualize the DNA content which becomes accessible upon permeabilisation of the cell membrane. The amount of PI-positive cells is directly correlated to the amount of dead cells, and measured using flow cytometry.

11B8 was not able to lyse either non-treated or lovastatin- or MβCD-mediated (Figure 14 and Figure 15C) cholesterol-depletion of Daudi cells via induction of CDC (negative control). As expected, both 2F2 and rituximab induced CDC of non-treated Daudi cells. Depletion of cholesterol from the cell membrane however detoriated the CDC-mediated lysis of Daudi cells induced by both these mAbs. Nevertheless, whereas the lysing potential of rituximab was abrogated, 2F2 could still efficiently lyse 50% of the cells. Data show a reduced CDC induction by anti-CD20 mAb 2F2 (Figure 15A) and rituximab (Figure 15B), but not by 11 B8 (Figure 15C) (negative control, EC50 could not be determined), of cells depleted for cholesterol via MβCD.

**Example 13: Sensitivity to CD20 mAb-induced CDC of cholesterol-treated WIL2-S cells detected via PI / FACS analysis**

Lovastatin-treated WIL2-S cells were in comparison to non-treated cells also less sensitive for CDC induction by 2F2 and rituximab (Figure 16A and Figure 16B). No effect was seen for 11B8 (Figure 16C). But as observed for binding, the CDC inducing capacity of 2F2 and rituximab could be fully restored upon replenishment of the cholesterol cell membrane levels. Addition of cholesterol to the culture of non-treated or cholesterol-depleted cells (as described in Example 4) reinstated the level of CDC induction of 2F2 and rituximab as determined by PI/FACS analysis.

In summary, depleting cholesterol from the cell membrane with either MβCD or lovastatin reduces the CDC-inducing capacity of anti-CD20 mAb. Replenishment of the cholesterol membrane content restores or further improves the lysing potential of the anti-CD20 mAb, depending on the B cell line used. This is observed for the type I anti-CD20 antibodies, i.e. antibodies having high CDC and ADCC activity, but low apoptosis activity, such as 2F2 and rituximab, but not for the type II anti-CD20 antibodies, i.e. antibodies having low or no CDC activity, but high ADCC and apoptosis activity, such as 11B8, which have no CDC lysing capacity at all.

**Example 14: CDC of cholesterol-depleted or -reconstituted CD20-expressing cells detected via MTT analysis**

CDC was induced by incubating B cells with anti-CD20 mAb (as described in Example 2). After 1-hour incubation (3-(4,5-dimethylthiazol-2-yl)-2,5-diphenyltetrazolium bromide (MTT) solution (5 mg/ml, Roche, Diagnostics, Almere, The Netherlands) was added to each well (flat-bottom microtiter plates; Nunc, Rochester, NY). The reaction was stopped after 4 hours of incubation at 37°C by the addition of 100 μl of 20% SDS (Roche Diagnostics, Almere, The Netherlands). After overnight incubation at 37°C the absorbance of the samples was measured at 550-525 nm using an Elisa reader (EL808x, Biotek instruments, Highland park, Vermont, USA). Cytotoxicity was expressed as a relative viability of the stimulated cells compared to cells incubated with medium only and was calculated as follows: relative viability =[(Ae-Ab)/(Ac-Ab)]* 100, where Ab is the background absorbance, Ae is experimental absorbance of the lysed cells and Ac is the absorbance of untreated cells. The viability was expressed as percentage survival in comparison to non-cholesterol depleted cells (control).

In addition to detection via PI/FACS analysis, the increased survival after CDC induction by rituximab of cholesterol-depleted RAJI cells was also observed using the MTT assay. Figure 17 confirms that 2F2-induced CDC was diminished when depleting cholesterol from the membrane of Daudi cells using increased concentrations of MβCD as detected by the MTT assay. Figure 18 also shows that depletion of cholesterol by either lovastatin or MβCD results in enhanced survival of RAJI cells when compared to non-treated cells. However, replenishment of cholesterol in depleted cells decreased the survival of the cells, and even further increased the capability of rituximab to lyse RAJI cells when compared to non-treated cells. Upon depletion of cholesterol with MβCD 2F2 was still more potent to lyse the cells in presence of complement than rituximab (Figure 19).

**Example 15: CDC of cholesterol-depleted CD20-expressing B cells detected via Alamar blue assay**

CDC was induced by incubating RAJI cells with anti-CD20 mAb (as described under Example 2). After 30 minutes of incubation, Alamar blue solution (Biosource, Camarillo, Ca, USA) was added to each well in the flat-bottomed microtiter plates (Nunc, Rochester, NY). The reaction was incubated for 5 hours at 37°C after which the conversion of the Alamar blue dye into a red fluorescent color was measured using the Synergy HT fluorometer (Biotek instruments, Highland park, Vermont, USA). Cell viability was detected as relative fluorescence units (RFI). Cytotoxicity was expressed as a relative viability of the stimulated cells compared to cells incubated with medium only and was calculated as follows: relative viability =[(RFIe-RFIb)/(RFIc-RFIb)]* 100, where RFIB is the background RFI, RFIe is experimental RFI of the lysed cells and RFIc is the absorbance of untreated cells. The viability was expressed as percentage survival in comparison to non-cholesterol depleted cells (control).

The enhanced survival of lovastatin-treated Daudi cells upon 2F2- and rituximab-induced CDC could also be detected using the Alamar blue assay (Figure 20). 2F2 was more potent in inducing cell lysis than rituximab or 11B8 (negative control). The survival was enhanced upon depletion of cholesterol out of the Daudi cell membrane. As observed using the PI/FACS analysis and MTT assay 2F2 was still capable of inducing CDC when treating cells with lovastatin.

**Example 16: Impaired rituximab-mediated CDC of RAJI-CD20high cells is dependent on cholesterol levels and not on disruption of lipid rafts.**

Binding of type I anti-CD20 mAbs has been shown to induce translocation of CD20 into cholesterol-rich microdomains, also referred to as lipid rafts, in the plasma membrane and this effect was associated with activation of CDC. Statins, by inhibiting cholesterol biosynthesis, have been shown to interfere with the formation of lipid rafts. Therefore, it was tested whether the impaired rituximab-mediated CDC induction was either dependent on the inability of CD20 to translocate into lipid rafts or dependent on the diminished cholesterol content. B cells were treated with chemicals either known to interfere with cholesterol synthesis, such as Berberine chloride, or known to interfere with cholesterol by disrupting its capacity to form lipid rafts, such as Fillipin III. Whereas Berberine chloride inhibits cholesterol synthesis by a HMG-CoAR-independent mechanism (cf. Kong W, et al. (2004) Berberine is a novel cholesterol-lowering drug working through a unique mechanism distinct from statins. Nat Med 10: 1344-1351), Filllipin III binds stoichometrically to cholesterol (cf. Smart EJ et al. (2002) Alterations in membrane cholesterol that affect structure and function of caveolae. Methods Enzymol 353: 131-139), thereby introducing additional charge that forces cholesterol molecules to distribute evenly within the plasma membrane and prevent lipid raft formation.

RAJI-CD20high cells were incubated with diluents (control) or a concentration range of either Fillipin III for 30 minutes or Berberine chloride for 24 hours. Cells ($1 \times 10^5$/well) were subjected to mAb-induced CDC by incubating the treated cells 60 minutes with 10 μg/ml rituximab in the presence of 10% complement active serum. The cytotoxic effects were measured in a MTT assay. 3-(4,5-Dimethylthiazol-2-yl)-2,5-diphenyltetrazolium bromide (MTT) solution (5 mg/ml, Roche, Diagnostics, Almere, The Netherlands) was added to each well in the flat-bottomed microtiter plates (Nunc, Rochester, NY). The reaction was stopped after 4 hours of incubation at 37°C by the addition of 100 μl of 20% SDS (Roche Diagnostics, Almere, The Netherlands). After overnight incubation at 37°C the absorbance of the samples was measured at 550-525 nm using an Elisa reader (EL808x, Biotek instruments, Highland park, Vermont, USA). Cytotoxicity was expressed as a relative viability of the stimulated cells compared to cells incubated with medium only and was calculated as follows: relative viability =[(Ae-Ab)/(Ac-Ab)]* 100, where Ab is the background absorbance, Ae is experimental absorbance of the lysed cells and Ac is the absorbance of untreated cells. The viability was expressed as percentage survival in comparison to non-cholesterol depleted cells (control).

A 24-hour incubation of RAJI-CD20high cells with Berberine chloride significantly abrogated the rituximab-mediated CDC whereas upon Fillipin III pre-incubation the cells remained sensitive to CDC induction with a single concentration of rituximab (Figure 21A).

The capacity of Fillipin III-treated or Berberine chloride-treated cells to bind B9E9 was determined by incubating treated cells with FITC-conjugated B9E9 for 30 minutes at 4°C. After washing twice with FACS buffer (PBS, 0.1% Bovine Serum Albumine, 0.02% Sodium Azide), cells were analyzed on a FACS Calibur (Becton Dickinson, Breda, The Netherlands).

As observed for induction of CDC, Berberine chloride-treated cells showed an impaired binding of B9E9 to RAJI-CD20high cells which was not observed for Fillipin III-treated cells (Figure 21 B).

Summarizing, the presence of cholesterol and not necessarily lipid rafts in the plasma membrane is critical for binding of anti-CD20 mAb as well as for anti-CD20-mediated CDC of lymphoma B cells.

**Example 17: Isolation of peripheral blood mononuclear cells (PBMC) from whole blood**

Blood was obtained from a donor (either from a healthy volunteer or from patients included in clinical trials related to 2F2). Informed consent was provided according to the Declaration of Helsinki. The blood was used to isolate peripheral blood mononuclear cells (PMBC) via sucrose gradient separation. Briefly, 10 ml of whole blood was supplemented with 20-25 ml PBS (Braun, Oss, Netherlands). 13 ml of Lymphocyte Separation Medium (Lonza, Verviers, Belgium) was pipetted carefully underneath the diluted whole blood solution. The solution was centrifuged at 2000 rpm for 20 minutes whereupon the interphase containing the PBMC was collected. The cells were washed twice in PBS, counted with trypan blue and suspended in test medium (RMPI 1640/ pen strep / 0.1% BSA) at a concentration of $2\text{-}4\text{x}10^6$ viable cells/ml. Cells were used to enrich B cells via negative depletion as described in Example 8.

**Example 18: Enrichment for B cells from PBMC via negative depletion**

PBMC (obtained as described under Example 17) were used to enrich for B cells using the Dynal B cell negative isolation kit (Dynal, Invitrogen, Carlsbad, CA) according to manufacturer's instruction. In brief, PBMC were washed and incubated for 20 minutes with mouse monoclonal antibodies directed against CD2, CD14, CD16, CD36, CD43 or CD235a at 4°C. Magnetic beads coated with human IgG4 against mouse monoclonal antibodies were added and the mixture was again incubated for 15 minutes at 4°C. After incubation mouse mAb-stained cells were separated from the mixture using a magnet. The remaining cells are considered as the B cell enriched population. B cell enrichment was checked using flow cytometry detecting expression of CD20, CD19 and CD21 (as B cell markers), and CD3 and goat-anti-mouse IgG (Jackson Immunoresearch Ltd, Suffolk, UK) of the PBMC and B cell enriched cell population (data not shown).

**Example 19: Anti-CD20 mAb binding and CDC induction of cholesterol-depleted freshly isolated B cells**

Human B cells were isolated from whole blood of a healthy volunteer (donor A) as described under Example 17 and Example 18. The cell population was enriched for B cells from 13% (in PBMC) up to 87% CD20-positive cells (data not shown).

To determine the effect of cholesterol depletion on the expression of cell surface proteins, surface expression of several B cell markers (CD20, CD19, CD21) and complement regulatory proteins (CD55 and CD59) was detected using flow immunofluorescence. Detection of CD3 (T-cell markers) served as a negative control. $1 \times 10^5$ cells were incubated with human mAb 20030730 AKI (2F2-FITC), and mouse mAb SJ25CI (CD19-PE), B-Ly4 (CD21-PE), IA10 (CD55-APC), p282 (CD59-FITC), TU36 HLA-DR-PE) and UCHT-1 (CD3-APC) (all from BD Pharmingen, Franklin Lakes, NJ) at saturating concentrations for 30 minutes at 4°C. After washing, cells were analyzed by flow cytometry. Identical stainings were performed on Daudi cells.

The B cell enriched cell population of donor A and Daudi cells were incubated with MβCD as described under Example 2 and cell surface protein expression and sensitivity for CDC was measured. As observed for Daudi cells, depletion of cholesterol out of the cell membrane of primary B cells resulted in a decreased binding of mAb to CD20 and CD19, whereas CD21 expression remained stable. Also the binding of the complement regulatory protein CD59 and of HLA-DR was diminished (Figure 22). As shown for Daudi cells, the B cell enriched cell population could be lysed by 2F2, but not by 11B8. When depleting cholesterol from the primary B cell population using MβCD, cell lysis was reduced using 2F2 and rituximab (Figure 23A). Although CDC induced by 2F2 showed a reduction upon MβCD treatment when using the mAb at low concentrations, approximately 75% of the primary B cell population was still killed under saturating conditions ($\geq 10\ \mu\text{g/ml}$). Under these conditions, no more than 20-25% of cell lysis was observed for rituximab when having cholesterol depleted from the cell membrane.

**Example 20: Cholesterol depletion impairs rituximab-mediated CDC as well as binding of anti-CD20 mAb to the surface of freshly isolated human lymphoma B cells**

To extend these observations the influence of cholesterol depletion was studied in freshly isolated human lymphoma B cells. Tumor cells of three mantle cell lymphomas and a small B cell lymphoma were isolated out of bone marrow (10 ml) aspirated from the hip. The cell suspension was diluted twice with PBS (final volume 20 ml). 3 ml of Histopaque-1077 (Sigma Aldrich) was pipetted into two conical centrifuge tubes. 10 ml of diluted bone marrow was slowly layered on the top of Histopaque layer. Probes were centrifuged (400 g, 15 minutes, 25°C) without brake. The white blood cell ring and plasma were isolated and washed twice with PBS. The leucocyte pellet was resuspeneded in 5 ml medium (RPMI or OptiMEM) and counted in a Burker chamber using Turk dye. Cells were incubated with either diluent or MβCD (10 mg/ml) for 30 minutes. Treated cells were analyzed for their capacity to bind CD20 mAb as well as sensitivity for rituximab-mediated CDC induction.

Cells were incubated with saturating amounts of FITC-conjugated B9E9 for 30 minutes at room temperature. After washing twice in FACS buffer CD20 mAb binding was analysed using a flow cytometer. In all four cases it was shown that 30 minutes incubation of B lymphoma cells with 10 mg/ml MβCD significantly decreased binding of B9E9 (Figure 24).

For the rituximab-mediated CDC human CD20-positive lymphoma B cells were incubated with either diluent or 10 mg/ml MβCD for 30 minutes. Then, equal numbers of cells ($1x10^5$/well) were incubated for 60 minutes with 400 μg/ml rituximab in the presence of 10% complement active serum. The cytotoxic effects were measured in a MTT assay. The survival of cells is presented as % of corresponding diluent- or MβCD-pretreated cells without rituximab. Accordingly to mAb binding, incubation with MβCD significantly decreased rituximab-mediated CDC against freshly isolated human lymphoma B cells (Figure 24).

**Example 21 : *In vivo* cholesterol depletion reduces CD20 expression on freshly isolated B cells of hypercholesterolemic patients treated with atorvastatin**

Further, the influence of cholesterol depletion in freshly isolated B cells of hypercholesterolemic patients treated with atorvastatin was studied. A small exploratory clinical trial was performed in which hypercholesterolemic patients (n=5) were treated with a single dose of 80 mg atorvastatin (taken in the evening) in order to reduce their cholesterol blood level and determine the effect on CD20 mAb binding on isolated B cells using flow cytometry. Approval of the study was obtained from the ethics review board of the Warsaw University Medical Center. Informed consent was provided according to the Declaration of Helsinki. Blood was drawn from patients in the morning after overnight abstention of food.

The cholesterol blood level was measured using automated dry chemistry system (Vitroz 250 System Chemistry (Ortho-Clinical Diagnostics, Johnson&Johnson, Piscataway, NJ). A starting cholesterol blood level of 190 mg/ml was required for patients to enroll in the clinical trial. At time of recruitment, patient I.D.#4 presented with a cholesterol blood concentration > 190 mg/ml, and as such was considered hypercholesterolemic. At time of recruitment the blood cholesterol level of patient I.D.#5 turned out to be below this cut off value, and thus it can be argued whether this patient should be considered hypercholesterolemic. As atovarstatin was already administered to this patient it was decided to include this patient in the trial. Cholesterol blood levels were subsequently measured on day 0 (pretreatment) and day 3 after treatment. In all five patients atorvastatin treatment induced a significant drop in blood cholesterol levels (Figure 25). The average cholesterol blood concentration of the five patients diminished with 17% ($\pm$ 4% SE) ($\pm$ 20 mg/ml SE) on day 0 to 172 mg/ml ($\pm$ 19 mg/ml SE).

CD21 is a protein expressed by B cells. Its expression has been shown unaffected upon cholesterol depletion of a B cell line (Daudi) and for freshly isolated B cell enriched cell population of two independent donors (Figure 26). Therefore, CD21 mAb binding is considered as an indicator for the amount of B cells present per staining. The MFI of CD20 mAb and CD21 mAb binding was identified by gating on viable cells in the lymphogate (FSC vs SSC), and subsequently on CD21+ cells when analyzing the MFI for the CD20 mAb. In order to be able to correlate the CD20 expression measured in between days CD20 mAb binding was quantitated relative to CD21 mAb binding within the same staining by calculating the ratio of MFI of CD20/CD21 mAb.

To determine CD20 and CD21 expression on B cells of hypercholesterolemic patients treated with atorvastatin PBMC were isolated out of whole blood drawn on day 0 (pre-treatment) and day 3 after treatment (as described in Example 7). Freshly isolated B cells were stained with different FITC-conjugated CD20 mAbs 2F2, 11 B8, B1 (Glaxo-SmithKline, Stevenage, United Kingdom) and PE-conjugated CD21 mAb (B-Ly4, BD Pharmingen, Franklin Lakes, NJ) by 30 minutes incubation at room temperature in the dark. Cells were washed and mAb binding intensity (MFI) was analysed using a FACS Calibur (Becton Dickinson, Breda, The Netherlands).

In all five patients three days after the single dose of atorvastatin a significant decrease of 18% ($\pm$ 4% SE, P=0.0010, paired t-test) in the average CD20 (2F2) expression (relative to CD21 (B-ly4) expression) on freshly isolated B cells was observed compared to day 0 (pretreatment) (Figure 27). When detecting CD20 expression using B1 a similar trend was observed. For three out of five patients a reduction in the CD20/CD21 MFI ratio was observed. The average decrease in B1-detected CD20/CD21 MFI ratio was 96% ($\pm$ 5% SE) (Figure 28). And, when detecting CD20 expression using CD20 mAb 11 B8 again a similar trend in reduction of the CD20/CD21 MFI ratio was observed for four out of five patients. The average decrease in 11B8-detected CD20/CD21 MFI ratio was 84% ($\pm$ 7% SE) (Figure 29). Thus, concurrent with a reduction in cholesterol blood levels a subsequent decrease in CD20 expression on freshly isolated B cells of hypercholesterolemic patients after statin treatment *in vivo* was observed. These data support that statin treatment lowers the CD20 expression on B cells *in vivo* which may affect the potency of the anti-CD20 mAb to exert immunotherapy in B cell driven diseases such as FL, CLL or RA.

**Example 22: Sensitivity to rituximab-induced antibody-dependent cellular cytotoxicty of cholesterol-depleted RAJI cells**

In the antibody-dependent cellular cytotoxicity (ADCC) assay target B cells are labelled with radioactive chromium ($^{51}$Cr), and incubated with antibody and effector cells. Upon lysis the target cells release $^{51}$Cr which can be measured by separation of the supernatant from the cells and quantification using a scintillation counter.

RAJI cells (obtained from ECACC, Porton Down, United Kingdom) served as target cells, and were cultured in RPMI 1640 supplemented with 10% heat-inactivated cosmic calf serum (CCS), 1 U/ml penicillin, 1 $\mu$g/ml streptomycin, and 4 mM L-glutamine (all from Invitrogen, Carlsbad, CA). RAJI cells were either non-treated or cholesterol-depleted using lovastatin (10 $\mu$M) as described in Example 3, and re-suspended in test medium to a concentration of $2 \times 10^6$ viable cells/ml.

RAJI cells were labeled with 20 $\mu$Ci $^{51}$Cr (Amersham Biosciences, Uppsala, Sweden) for 2 hours. After extensive washing in medium, the cells were adjusted to $2 \times 10^5$ cells/ml. PBMCs were used as effector cells (50 $\mu$l, see Example 17 for isolation of PBMCs), a concentration curve of rituximab (50 $\mu$l) and medium (50 $\mu$l) were added to round-bottom microtiter plates (Greiner Bio-One GmbH, Frickenhausen, Germany). Assays were started by adding $^{51}$Cr-labeled RAJI cells (50 $\mu$l) giving a final volume of 200 $\mu$l. For isolated effector cells, containing 20% NK cells, an effector to target (E:T) ratio of 100:1 was used. After incubation (4 hours, 37°C), assays were stopped by centrifugation, and $^{51}$Cr release from triplicates was measured in counts per minute (cpm) in a scintillation counter. Percentage of cellular cytotoxicity was calculated using the following formula:

$$\% \ \text{specific lysis} = (\text{experimental cpm} - \text{basal cpm})/(\text{maximal cpm} - \text{basal cpm}) \times 100$$

with maximal $^{51}$Cr release determined by adding TritonX-100 (5% final concentration) to target cells, and basal release measured in the absence of sensitizing antibodies and effector cells.

A concentration-dependent induction of ADCC-mediated lysis of RAJI cells in the presence of rituximab was observed. However, cholesterol-depleted cells (lovastatin) showed a reduced ADCC-mediated lysis of RAJI cells when compared to the non-treated cells (Figure 30). The $EC_{50}$ value for rituximab-mediated ADCC increased $\sim$ 4-fold for the lovastatin-treated cells ($EC_{50}$ control: 0.024 $\mu$g/ml; $EC_{50}$ lovastatin: 0.082 $\mu$g/ml).

**Example 23: Formulation of ofatumumab (2F2)**

The following 20 mg/ml aqueous formulation of ofatumumab is prepared by standard procedures:

| Ingredient | Quantity per ml | Function |
| --- | --- | --- |
| Ofatumumab drug substance | 20 mg | Active ingredient |
| Sodium Citrate USP/EP | 8.549 mg | Buffering and stabilizing agent |
| Citric Acid USP/EP | 0.195 mg | Buffering and stabilizing agent |
| Sodium Chloride USP/EP | 5.844 mg | Isotonic agent |
| Water for injection USP/EP q.s. to | 1 ml | Solvent |

**Example 24: Withdrawal of statin treatment in FL patients prior to ofatumumab administration**

Patients suffering from FL on statin therapy are withdrawn from statin therapy 3 months prior to the first administration of ofatumumab. Ofatumumab is administered by intravenous infusion according to the following regimen: 8 weekly infusions of ofatumumab, the first infusion of 300 mg of ofatumumab and the subsequent infusions of each 1000 mg of ofatumumab. Two to three months after the last administration of ofatumumab the patients are resuming statin therapy.

**Example 25: Monitoring the cholesterol blood level**

The cholesterol level may be monitored in the patients to evaluate the need for increasing the cholesterol level prior to administration with ofatumumab. The cholesterol blood level should preferably be above 130 mg/ml, such as above 160 mg/ml, 190 mg/ml or 220 mg/ml during administration of ofatumumab.

**Claims**

1. An antibody capable of mediating CDC, which specifically binds to CD20, and which comprises a $V_H$ CDR1 of SEQ ID NO:3, a $V_H$ CDR2 of SEQ ID NO:4, a $V_H$ CDR3 of SEQ ID NO:5, a $V_L$ CDR1 of SEQ ID NO:6, a $V_L$ CDR2 of SEQ ID NO:7 and a $V_L$ CDR3 sequence of SEQ ID NO:8, for use in the treatment of a disease or disorder associated with said antigen, wherein the disease or disorder is selected from B cell lymphoma, B cell leukemia or an autoimmune disease, and wherein antibody-induced CDC has a beneficial effect on said disease or disorder, wherein the antibody is to be administered to a subject undergoing therapy with a cholesterol-lowering agent, such as a statin, and wherein the subject is withdrawn from treatment with the cholesterol-lowering agent prior to the administration of the antibody.

2. An antibody for use according to claim 1, wherein the subject is withdrawn from treatment with the cholesterol-lowering agent for a period of from at least 7 days, such as 14 days, 30 days, 45 days, 60 days or 90 days prior to the first administration of the antibody until at least 7 days, such as 14 days, 30 days, 45 days, 60 days or 90 days after the last administration of the antibody in a regimen.

3. An antibody for use according to any one of claims 1-2, **characterized in that** the antibody is a human monoclonal antibody.

4. An antibody for use according to according to any one of the preceding claims 1-3, wherein the disease is selected from follicular lymphoma (FL), chronic lymphocytic leukemia (CLL), diffuse large B-cell lymphoma (DLBCL), rheumatoid arthritis (RA), systemic lupus erythematosus (SLE), multiple sclerosis (MS), Sjögren's syndrome (SS), and chronic obstructive pulmonary disease (COPD).

5. An antibody for use according to any one of the claims 1-4, **characterized in that** the antibody against CD20 has human heavy chain and human light chain variable regions comprising the amino acid sequences as set forth in SEQ ID NO:1 and SEQ ID NO:2, respectively; or amino acid sequences which are at least 95% homologous, and more preferably at least 98%, or at least 99% homologous to the amino acid sequences as set forth in SEQ ID NO:1 and SEQ ID NO:2, respectively.

6. An antibody for use according to any one of the preceding claims, wherein the treatment comprises one or more further therapeutic agents.

## Patentansprüche

1. Antikörper, welcher geeignet ist, CDC zu vermitteln, welcher spezifisch an CD20 bindet und welcher eine $V_H$ CDR1 gemäß SEQ ID NO:3, ein $V_H$ CDR2 gemäß SEQ ID NO:4, eine $V_H$ CDR3 gemäß SEQ ID NO:5, eine $V_L$ CDR1 gemäß SEQ ID NO:6, eine $V_L$ CDR2 gemäß SEQ ID NO:7 und eine $V_L$ CDR3-Sequenz gemäß SEQ ID NO:8 umfasst, zur Verwendung in der Behandlung einer Krankheit oder Störung, welche mit dem Antigen assoziiert ist, wobei die Krankheit oder Störung ausgewählt ist aus B-Zell-Lymphom, B-Zell-Leukämie oder einer Autoimmunkrankheit, und wobei die Antikörper-induzierte CDC einen nutzbringenden Effekt auf diese Krankheit oder Störung hat, wobei der Antikörper einem Patienten zu verabreichen ist, welcher sich einer Therapie mit einem Cholesterin-senkenden Stoff, wie etwa einem Statin, unterzieht und wobei der Patient die Behandlung mit dem Cholesterin-senkenden Stoff vor der Verabreichung des Antikörpers beendet.

2. Antikörper zur Verwendung gemäß Anspruch 1, wobei der Patient die Behandlung mit dem Cholesterin-senkenden Stoff für einen Zeitraum von mindestens 7 Tagen, etwa 14 Tagen, 30 Tagen, 45 Tagen, 60 Tagen oder 90 Tagen beendet vor der ersten Verabreichung des Antikörpers bis mindestens 7 Tage, wie etwa 14 Tage, 30 Tage, 45 Tage, 60 Tage oder 90 Tage nach der letzten Verabreichung des Antikörpers in einem Verabreichungsplan.

3. Antikörper zur Verwendung gemäß einem der Ansprüche 1-2, charakterisiert dadurch, dass der Antikörper ein menschlicher monoklonaler Antikörper ist.

4. Antikörper zur Verwendung gemäß einem der vorherigen Ansprüche 1-3, wobei die Krankheit ausgewählt ist aus follikulärem Lymphom (FL), chronischer Lymphozytenleukämie (CLL), diffusem großem B-Zell-Lymphom (DLBCL), rheumatoider Arthritis (RA), systemischem Lupus erythematodes (SLE), Multipler Sklerose (MS), Sjögren-Syndrom (SS) und chronischobstruktiver Lungenerkrankung (COPD).

5. Antikörper zur Verwendung gemäß einem der Ansprüche 1-4, charakterisiert dadurch, dass der Antikörper gegen CD20 variable Regionen der menschlichen schweren Kette und menschlichen leichten Kette hat, umfassend die Aminosäuresequenzen wie in SEQ ID NO:1 und SEQ ID NO:2 gezeigt oder Aminosäuresequenzen, welche mindestens 95% homolog und weiter bevorzugt mindestens 98% oder mindestens 99% homolog zur Aminosäuresequenz wie in SEQ ID NO:1 bzw. SEQ ID NO:2 gezeigt sind.

6. Antikörper zur Verwendung gemäß einem der vorherigen Ansprüche, wobei die Behandlung ein oder mehrere zusätzliche therapeutische Agenzien umfasst.

## Revendications

1. Anticorps capable de médier une fonction CDC, qui se lie spécifiquement à CD20, et qui comprend une région CDR1 de région $V_H$ de Séquence N° 3, une région CDR2 de région $V_H$ de Séquence N° 4, une région CDR3 de région $V_H$ de Séquence N° 5, une région CDR1 de région $V_L$ de Séquence N° 6, une région CDR2 de région $V_L$ de Séquence N° 7, et une région CDR3 de région $V_L$ de Séquence N° 8, pour utilisation dans le traitement d'une maladie ou d'un trouble associé audit antigène, laquelle maladie ou lequel trouble est choisi parmi un lymphome à lymphocytes B, une leucémie à lymphocytes B et une maladie auto-immune, et laquelle fonction CDC induite par anticorps a un effet bénéfique sur ladite maladie ou ledit trouble, lequel anticorps est à administrer à un sujet subissant une thérapie par hypo-cholestérolémiant, telle une statine, étant entendu qu'avant l'administration de l'anticorps, on arrête de traiter le sujet par l'hypocholestérolémiant.

2.  Anticorps pour utilisation conforme à la revendication 1, dans laquelle on arrête de traiter le sujet par l'hypocholestérolémiant pendant une période allant d'au moins 7 jours, comme 14, 30, 45, 60 ou 90 jours, avant la première administration de l'anticorps jusqu'à au moins 7 jours, comme 14, 30, 45, 60 ou 90 jours, après la dernière administration de l'anticorps dans le programme.

3.  Anticorps pour utilisation conforme à l'une des revendications 1 et 2, **caractérisé en ce que** l'anticorps est un anticorps monoclonal humain.

4.  Anticorps pour utilisation conforme à l'une des revendications 1 à 3 précédentes, dans laquelle la maladie est choisie parmi les suivantes : lymphome folliculaire (FL), leucémie lymphocytaire chronique (CLL), lymphome diffus à grands lymphocytes B (DLBCL), polyarthrite rhumatoïde (RA), lupus érythémateux aigu disséminé (SLE), sclérose en plaques (MS), syndrome de Sjögren (SS), et bronchopneumopathie chronique obstructive (COPD).

5.  Anticorps pour utilisation conforme à l'une des revendications 1 à 4, **caractérisé en ce que** l'anticorps dirigé contre CD20 comporte des régions variables de chaîne lourde humaines et des régions variables de chaîne légère humaines comprenant les séquences d'acides aminés indiquées respectivement dans la Séquence N° 1 et la Séquence N° 2, ou des séquences d'acides aminés qui sont homologues, à un degré d'au moins 95 %, et de préférence d'au moins 98 % ou d'au moins 99 %, aux séquences d'acides aminés indiquées respectivement dans la Séquence N° 1 et la Séquence N° 2.

6.  Anticorps pour utilisation conforme à l'une des revendications précédentes, dans laquelle le traitement comporte un ou plusieurs agent(s) thérapeutique(s) supplémentaire(s).

Figure 1A

## Figure 1B

**Figure 2**

**Figure 3A**

**Figure 3B**

## Figure 4A

## Figure 4B

## Figure 5A

## Figure 5B

## Figure 6A

## Figure 6B

## Figure 7A

## Figure 7B

**Figure 7C**

## Figure 8

## Figure 9

## Figure 10

## Figure 11A

## Figure 11B

Merged    Merged

## Figure 11C

## Figure 12

## Figure 13A

## Figure 13B

## Figure 13C

## Figure 14

## Figure 15A

## Figure 15B

**Figure 15C**

## Figure 16A

## Figure 16B

## Figure 16C

## Figure 17

## Figure 18

## Figure 19

## Figure 20

## Figure 21A

## Figure 21B

## Figure 22A

## Figure 22B

**Figure 23A**

**Figure 23B**

**Figure 24**

# Figure 24 continued

**Figure 25A**

**Figure 25B**

**Figure 26**

# Figure 27

**Figure 28**

## Figure 29

## Figure 30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007059782 A
- EP 404097 A
- WO 9311161 A
- US 4816567 A
- EP 125023 A
- EP 171496 A
- EP 173494 A
- WO 8601533 A
- EP 184187 A
- WO 8702671 A
- US 5545806 A
- US 5569825 A
- US 5625126 A
- US 5633425 A
- US 5789650 A
- US 5877397 A
- US 5661016 A
- US 5814318 A
- US 5874299 A
- US 5770429 A, Lonberg and Kay
- US 5545807 A, Surani
- WO 9824884 A
- WO 9425585 A
- WO 931227 A
- WO 9222645 A
- WO 9203918 A
- WO 0109187 A
- WO 0243478 A
- WO 2004035607 A

**Non-patent literature cited in the description**

- **POLYAK MJ et al.** *Leukemia,* 2003, vol. 17, 1384-1389
- **JANAS E et al.** *Clinical and Experimental Immunology,* 2005, vol. 139, 439-446
- **CRAGG MS et al.** *Curr Dir Autoimmun,* 2005, vol. 8, 140-174
- Fundamental Immunology. Raven Press, 1989
- **CHOTHIA ; LESK.** *J. Mol. Biol.,* 1987, vol. 196, 901-917
- **KABAT et al.** Sequences of Proteins of Immunological Interest. National Institutes of Health, 1991
- **WARD et al.** *Nature,* 1989, vol. 341, 544-546
- **HOLT et al.** *Trends Biotechnol.,* November 2003, vol. 21 (11), 484-90
- **REVETS et al.** *Expert Opin Biol Ther.,* January 2005, vol. 5 (1), 111-24
- **BIRD et al.** *Science,* 1988, vol. 242, 423-426
- **HUSTON et al.** *PNAS USA,* 1988, vol. 85, 5879-5883
- *PNAS USA,* 1993, vol. 90 (14), 6444-6448
- **MORRISON et al.** *PNAS USA,* 1984, vol. 81, 6851-6855
- **CABILLY et al.** *PNAS USA,* 1984, vol. 81, 3273-3277
- **BOULIANNE E.** *Nature,* 1984, vol. 312, 643-646
- **NEUBERGER et al.** *Nature,* 1985, vol. 314, 268-270
- **SAHAGAN et al.** *J. Immunol.,* 1986, vol. 137, 1066-1074
- **LIU et al.** *PNAS USA,* 1987, vol. 84, 3439-3443
- **SUN et al.** *PNAS USA,* 1987, vol. 84, 214-218
- **BETTER et al.** *Science,* 1988, vol. 240, 1041-1043
- **HARLOW et al.** Antibodies: A Laboratory Manual. Cold Spring Harbor Laboratory Press, 1988
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **TAYLOR L et al.** *Nucleic Acids Research,* 1992, vol. 20, 6287-6295
- **CHEN J et al.** *International Immunology,* 1993, vol. 5, 647-656
- **TUAILLON et al.** *J. Immunol.,* 1994, vol. 152, 2912-2920
- **LONBERG N et al.** *Nature,* 1994, vol. 368 (6474), 856-859
- **LONBERG N.** *Handbook of Experimental Pharmacology,* 1994, vol. 113, 49-101
- **TAYLOR LET.** *International Immunology,* 1994, vol. 6, 579-591
- **LONBERG N ; HUSZAR D.** *Intern. Rev. Immunol.,* 1995, vol. 13, 65-93
- **HARDING F ; LONBERG N.** *Ann. N.Y. Acad. Sci,* 1995, vol. 764, 536-546

- **FISHWILD D et al.** *Nature Biotechnology,* 1996, vol. 14, 845-851
- **GUO Y et al.** *Cancer Res,* 2008, vol. 68 (7), 2400-2408
- **LORRAINE JOHNSTON.** Non-Hodgkin's Lymphomas: Making sense of Diagnosis, Treatment, and Options. O'Reilly and Associates, Inc, 1999
- **CROWE et al.** *Proc Natl Acad Sci USA,* 15 February 1994, vol. 91 (4), 1386-1390
- **YANG et al.** *PNAS USA,* 2003, vol. 100 (12), 6934-6939
- **BERGE, S.M. et al.** *J. Pharm. Sci.,* 1977, vol. 66, 1-19
- **BAEK et al.** *Methods Enzymol.,* 2003, vol. 362, 240-9
- **NIGAVEKAR et al.** *Pharm Res.,* 2004, vol. 21 (3), 476-83
- **POWELL et al.** Compendium of excipients for parenteral formulations. *PDA J Pharm Sci Technol.,* 1998, vol. 52, 238-311
- Sustained and Controlled Release Drug Delivery Systems. Marcel Dekker, Inc, 1978
- **STREJAN et al.** *J. Neuroimmunol.,* 1984, vol. 7, 27
- **MCTAGGART SJ.** Isoprenylated proteins. *Cell Mol Life Sci,* 2006, vol. 63, 255-267
- **LIAO JK ; LAUFS U.** Pleiotropic effects of statins. *Annu Rev Pharmacol Toxicol,* 2005, vol. 45, 89-118
- **ALEGRET M ; SILVESTRE JS.** Pleiotropic effects of statins and related pharmacological experimental approaches. *Methods Find Exp Clin Pharmacol,* 2006, vol. 28, 627-656
- **ITO MK ; TALBERT RL ; TSIMIKAS S.** Statin-associated pleiotropy: possible effects beyond cholesterol reduction. *Pharmacotherapy,* 2006, vol. 26, 85S-97S
- **KONG W et al.** Berberine is a novel cholesterol-lowering drug working through a unique mechanism distinct from statins. *Nat Med,* 2004, vol. 10, 1344-1351
- **SMART EJ et al.** Alterations in membrane cholesterol that affect structure and function of caveolae. *Methods Enzymol,* 2002, vol. 353, 131-139